# EUROPEAN PATENT SPECIFICATION

(11) **EP 2 480 089 B1**
(45) Date of publication and mention of the grant of the patent: **23.03.2016**
(21) Application number: 10771177.2
(22) Date of filing: 17.09.2010
(51) Int. Cl.: C07K 14/705

(54) **INACTIVATED MICROORGANISMS CONTAINING DOUBLE -STRAND RNA MOLECULES (DSRNA), THEIR USE AS PESTICIDES AND METHODS FOR THEIR PREPARATION**
INAKTIVIERTE MIKROORGANISMEN MIT DOPPELSTRÄNGIGEN RNA-MOLEKÜLEN (DSRNA), IHRE VERWENDUNG ALS PESTIZIDE UND HERSTELLUNGSVERFAHREN
MICRO-ORGANISMES INACTIVÉS CONTENANT DES MOLÉCULES D'ARN DOUBLE BRIN (DSARN), LEUR UTILISATION COMME PESTICIDES ET LEURS PROCÉDÉS DE PRÉPARATION

(30) Priority: 25.09.2009 IT MI20091649
(43) Date of publication of application: 01.08.2012
(73) Proprietor: Arterra Bioscience S.R.L., 80142 Napoli (IT)
(72) Inventor: TORTORA, Assunta, 84016 Pagani (SA) (IT); GRIMALDI, Maria Rosaria, 80010 Quarto (NA) (IT); RUGGIERO, Alessandra, 80128 Napoli (IT); FILIPPINI, Lucio, 28100 Novara (IT); APONE, Fabio, 80128 Napoli (IT); COLUCCI, Maria Gabriella, 80142 Napoli (IT)
(74) Representative: Finetti, Claudia
(86) International application number: PCT/IB2010/002363
(87) International publication number: WO 2011/036536

(56) References cited:
- WO-A2-03/052078
- ZHENG ET AL: "Cloning and characterization of the pheromone biosynthesis activating neuropeptide receptor gene in Spodoptera littoralis larvae", GENE, ELSEVIER, AMSTERDAM, NL, vol. 393, no. 1-2, 11 April 2007 (2007-04-11), pages 20-30, XP22104630, ISSN: 0378-1119
- TIAN HONGGANG ET AL: "Developmental control of a lepidopteran pest Spodoptera exigua by ingestion of bacteria expressing dsRNA of a non-midgut gene", PLOS ONE, vol. 4, no. 7, E6225, 1 January 2009 (2009-01-01), pages 1-13, XP002601508,
- BAUM JAMES A ET AL: "Control of coleopteran insect pests through RNA interference.", November 2007 (2007-11), NATURE BIOTECHNOLOGY NOV 2007 LNKD- PUBMED:17982443, VOL. 25, NR. 11, PAGE(S) 1322 - 1326, XP002532086, ISSN: 1087-0156 the whole document
- PRICE D R G ET AL: "RNAi-mediated crop protection against insects", TRENDS IN BIOTECHNOLOGY, ELSEVIER PUBLICATIONS, CAMBRIDGE, GB LNKD- DOI:10.1016/J.TIBTECH.2008.04.004, vol. 26, no. 7, 1 July 2008 (2008-07-01), pages 393-400, XP022757296, ISSN: 0167-7799 [retrieved on 2008-05-22]
- AUERSWALD L ET AL: "Structural, functional, and evolutionary characterization of novel members of the allatostatin receptor family from insects.", BIOCHEMICAL AND BIOPHYSICAL RESEARCH COMMUNICATIONS 13 APR 2001, vol. 282, no. 4, 13 April 2001 (2001-04-13), pages 904-909, ISSN: 0006-291X
- WANG SHUZHEN ET AL: "Discovery of a small molecule targeting SET-PP2A interaction to overcome BCR-ABLT315I mutation of chronic myeloid leukemia.", ONCOTARGET 20 MAY 2015, vol. 6, no. 14, 20 May 2015 (2015-05-20), pages 12128-12140, ISSN: 1949-2553
- GUERGNON JULIEN ET AL: "Use of penetrating peptides interacting with PP1/PP2A proteins as a general approach for a drug phosphatase technology", MOLECULAR PHARMACOLOGY, AMERICAN SOCIETY FOR PHARMACOLOGY AND EXPERIMENTAL THERAPEUTICS, US, vol. 69, no. 4, 1 April 2006 (2006-04-01), pages 1115-1124, XP002539070, ISSN: 0026-895X, DOI: 10.1124/MOL.105.019364

### SEQUENCE LISTING

<110> Arterra Bioscience S.r.l.
<120> Inactivated microrganisms containing double strand RNA molecules (dsRNA), their use as pesticides and methods for the preparation thereof
<130> PCT99060
<150> MI2009A001649
   <151> 2009-09-25
<160> 26
<170> PatentIn version 3.5
<210> 1
   <211> 1371
   <212> DNA
   <213> Artificial
<220>
   <223> Allatostatin C receptor sequence AlstCR
<400> 1
   catattgctg tgtacgtggt gacgcaggtg ttgtacgcgc tcgtctgcgt ggtggggctg      60
   ctcggcaaca ccctcgtcat ctacgtcgtg ctgcgctact ccaagatgca gaccgtcacc     120
   aacatgtaca tcgtgaacct cgccatcgct gacgaatgct ttcttatcgg catcccattc     180
   ctgatcacaa cgatgtctct tcgtgcgtgg ccgtttggac gcttcatgtg taaggcctac     240
   atgatatcta ccggaatcaa ccagttcacc agctctatat tcttgtgtat aatgagtgcg     300
   gatcgataca tagccgtgtg tcatccgata gccgcgccgc gactgcggac gccattcgtc     360
   tctagagtgg tgtcagctgc agcatggaca gcgtcagctt tggtgatgac gcctatattt     420
   atgtacacga cactcatacc aacaccccat ggtctgtcgt gcaatatagt gtggcccgag     480
   caagagttca ataaaggcca gacgtccttc actctgtact cgttcgctct cggtttcgcc     540
   gcaccactca cccttatctt cgtgttttac tgtctcgtga ttcgcaaact gaagactgtc     600
   ggccctaaga acaaatctaa agagaaaaag cgatcacaca gaaaagtaac caaattagtg     660
   ctgactgtca ttgctgtgta cgtgctgtgc tggctgccgt actgggcgtt ccaggtagct     720
   ctcatttact ctccaccgaa cgagtgcgcc agccagatca ctatcaccgt ttttctggta      780
   gccgcgtgct tcagttacag taactctgcc atgaacccaa ttctttacgc ctttctttct     840
   gacaacttca agaaaagttt cctaaaggcc tgcacctgtg cagctgggaa agatgtgaat     900
   gcaactcttc atgtggagaa cagtgtgata ccgaggaagc ggggaggtgg caccgcgctg     960
   ctggaggagg acacgctgat cgtgtccatc gactagagta gtaagactgt cggacgggac    1020
   gccgtcggcc gcagagactc gagccctgtc tctcggccgc cgagcagccc acgggtccag    1080
   ttttgtaccg gttcaagttt taaatattag ttgtaaggtg tgcggtgtgt gagatatgtg    1140
   atggagttgt tctgtttgtg tataaagtca aaattgtatg gagtcggttc ggcctagggt    1200
   tgtaacgatg aatttactaa aatgtaattt tttatcgcat agcgactgta aggttactgt    1260
   ttgtatactg tgtatttaag gtgtaaaaaa ttgtacatat cgtgtaaata tttgtgaact    1320
   tgtcataatg tcacagttgt aataaaatta cgttcgtgaa aaaaaaaaaa a             1371
<210> 2
   <211> 331
   <212> PRT
   <213> Artificial
<220>
   <223> Protein sequence allatostatin receptor C
<400> 2
   His Ile Ala Val Tyr Val Val Thr Gln Val Leu Tyr Ala Leu Val Cys
   1               5                   10                  15
   Val Val Gly Leu Leu Gly Asn Thr Leu Val Ile Tyr Val Val Leu Arg
               20                  25                  30
   Tyr Ser Lys Met Gln Thr Val Thr Asn Met Tyr Ile Val Asn Leu Ala
           35                  40                  45
   Ile Ala Asp Glu Cys Phe Leu Ile Gly Ile Pro Phe Leu Ile Thr Thr
       50                  55                  60
   Met Ser Leu Arg Ala Trp Pro Phe Gly Arg Phe Met Cys Lys Ala Tyr
   65                  70                  75                  80
   Met Ile Ser Thr Gly Ile Asn Gln Phe Thr Ser Ser Ile Phe Leu Cys
                   85                  90                  95
   Ile Met Ser Ala Asp Arg Tyr Ile Ala Val Cys His Pro Ile Ala Ala
               100                 105                 110
   Pro Arg Leu Arg Thr Pro Phe Val Ser Arg Val Val Ser Ala Ala Ala
           115                 120                 125
   Trp Thr Ala Ser Ala Leu Val Met Thr Pro Ile Phe Met Tyr Thr Thr
       130                 135                 140
   Leu Ile Pro Thr Pro His Gly Leu Ser Cys Asn Ile Val Trp Pro Glu
   145                 150                 155                 160
   Gln Glu Phe Asn Lys Gly Gln Thr Ser Phe Thr Leu Tyr Ser Phe Ala
                   165                 170                 175
   Leu Gly Phe Ala Ala Pro Leu Thr Leu Ile Phe Val Phe Tyr Cys Leu
               180                 185                 190
   Val Ile Arg Lys Leu Lys Thr Val Gly Pro Lys Asn Lys Ser Lys Glu
           195                 200                 205
   Lys Lys Arg Ser His Arg Lys Val Thr Lys Leu Val Leu Thr Val Ile
                   210                 215                 220
   Ala Val Tyr Val Leu Cys Trp Leu Pro Tyr Trp Ala Phe Gln Val Ala
   225                 230                 235                 240
   Leu Ile Tyr Ser Pro Pro Asn Glu Cys Ala Ser Gln Ile Thr Ile Thr
                   245                 250                 255
   Val Phe Leu Val Ala Ala Cys Phe Ser Tyr Ser Asn Ser Ala Met Asn
               260                 265                 270
   Pro Ile Leu Tyr Ala Phe Leu Ser Asp Asn Phe Lys Lys Ser Phe Leu
           275                 280                 285
   Lys Ala Cys Thr Cys Ala Ala Gly Lys Asp Val Asn Ala Thr Leu His
       290                 295                 300
   Val Glu Asn Ser Val Ile Pro Arg Lys Arg Gly Gly Gly Thr Ala Leu
   305                 310                 315                 320
   Leu Glu Glu Asp Thr Leu Ile Val Ser Ile Asp
                   325                 330
<210> 3
   <211> 534
   <212> DNA
   <213> Artificial
<220>
   <223> Adipokinetic hormone receptor AKHR nucleotidic sequence
<400> 3
   gacctgatgt gccgagtcat gatgttcacc aggaccttcg gactgtacct ctccagcttc      60
   gtgcttatct gtatagctgt tgatagatac tacgccattt taaagccttt gaacgtgaca     120
   tgggaggcgc gcgtgcgcag agcgctgatt atcgcgtggg tgtgcgccgg cctcgccagc     180
   ctgccgcaga gcttcatatt ccacgtagag gagcatcctg atgttaaggg gtactaccag     240
   tgcgtgtcgt acgggtcgtt gccgacggag tgccacgagt ttgcgtactt cttggtaaac     300
   atgatcctca tgtacgtcat ccctctgctg tccacgctgt actgctcctc ggcagctttg     360
   ttggaaatca ttcggagatc taacacttct aatgataaga tgcgacggag cggcgtgggt     420
   atactcgggc gggcgcgggc tcggacactc aagatgacag tcactatagt tctagtcttt     480
   tttacatgtt ggtcacccta ctactgttat tgtctgtggt attggatcga caag           534
<210> 4
   <211> 178
   <212> PRT
   <213> Artificial
<220>
   <223> Protein sequence AKHR receptor
<400> 4
   Asp Leu Met Cys Arg Val Met Met Phe Thr Arg Thr Phe Gly Leu Tyr
   1               5                   10                  15
   Leu Ser Ser Phe Val Leu Ile Cys Ile Ala Val Asp Arg Tyr Tyr Ala
               20                  25                  30
   Ile Leu Lys Pro Leu Asn Val Thr Trp Glu Ala Arg Val Arg Arg Ala
           35                  40                  45
   Leu Ile Ile Ala Trp Val Cys Ala Gly Leu Ala Ser Leu Pro Gln Ser
       50                  55                  60
   Phe Ile Phe His Val Glu Glu His Pro Asp Val Lys Gly Tyr Tyr Gln
   65                  70                  75                  80
   Cys Val Ser Tyr Gly Ser Leu Pro Thr Glu Cys His Glu Phe Ala Tyr
                   85                  90                  95
   Phe Leu Val Asn Met Ile Leu Met Tyr Val Ile Pro Leu Leu Ser Thr
               100                 105                 110
   Leu Tyr Cys Ser Ser Ala Ala Leu Leu Glu Ile Ile Arg Arg Ser Asn
           115                 120                 125
   Thr Ser Asn Asp Lys Met Arg Arg Ser Gly Val Gly Ile Leu Gly Arg
       130                 135                 140
   Ala Arg Ala Arg Thr Leu Lys Met Thr Val Thr Ile Val Leu Val Phe
   145                 150                 155                 160
   Phe Thr Cys Trp Ser Pro Tyr Tyr Cys Tyr Cys Leu Trp Tyr Trp Ile
                   165                 170                 175
   Asp Lys
<210> 5
   <211> 30
   <212> DNA
   <213> Artificial
<220>
   <223> AlstCR Forward primer
<400> 5
   gaagtgtctt tctctagcta tatcggatgc 30
<210> 6
   <211> 30
   <212> DNA
   <213> Artificial
<220>
   <223> AlstCR Reverse primer
<400> 6
   atgcgcagca atgcgtagca atgcgctctt 30
<210> 7
   <211> 24
   <212> DNA
   <213> Artificial
<220>
   <223> AKHR Forward primer
<400> 7
   gacctgatgt gcacgacagt catg 24
<210> 8
   <211> 22
   <212> DNA
   <213> Artificial
<220>
   <223> AKHR Reverse primer
<400> 8
   gtcagatcca agtaccacag ag 22
<210> 9
   <211> 1086
   <212> DNA
   <213> Spodoptera littoralis
<220>
   <221> CDS
   <222> (1)..(1086)
   <223> Allatostatin A receptor encoding sequence
<400> 9
   atg gcg tcg act gaa gac gaa ttt agg agc ata tgc ctt aac tta acc      48
   Met Ala Ser Thr Glu Asp Glu Phe Arg Ser Ile Cys Leu Asn Leu Thr
   1               5                   10                  15
   agt gag gag gct atg ctt gga gga tgt aac tac agc agc gaa tat gaa      96
   Ser Glu Glu Ala Met Leu Gly Gly Cys Asn Tyr Ser Ser Glu Tyr Glu
               20                  25                  30
   aat gga gaa ctg ttg gag aag gtc gtc tcg agg gtt gtg ccc atc ttc      144
   Asn Gly Glu Leu Leu Glu Lys Val Val Ser Arg Val Val Pro Ile Phe
           35                  40                  45
   ttt ggc ttc ata ggc atc gtt gga tta gtt ggc aat gct ttg gtc gtc      192
   Phe Gly Phe Ile Gly Ile Val Gly Leu Val Gly Asn Ala Leu Val Val
       50                  55                  60
   tta gtg gtc gct gcc aac cct ggc atg cgg tcc act acc aac ctg ctc      240
   Leu Val Val Ala Ala Asn Pro Gly Met Arg Ser Thr Thr Asn Leu Leu
   65                  70                  75                  80
   atc ata aac ttg gct gtc gct gac ttg ctc ttt gtg atc ttc tgc gtt      288
   Ile Ile Asn Leu Ala Val Ala Asp Leu Leu Phe Val Ile Phe Cys Val
                   85                  90                  95
   cca ttc act gct acc gac tat gtc atg cca cga tgg ccc ttc gga gac      336
   Pro Phe Thr Ala Thr Asp Tyr Val Met Pro Arg Trp Pro Phe Gly Asp
               100                 105                 110
   tgg tgg tgc aag gtt gtc cag tac ttc att gta gtc act gct cat gcg      384
   Trp Trp Cys Lys Val Val Gln Tyr Phe Ile Val Val Thr Ala His Ala
           115                 120                 125
   tcc gtc tac act cta gta ctc atg tct cta gac agg ttc atg gct gta      432
   Ser Val Tyr Thr Leu Val Leu Met Ser Leu Asp Arg Phe Met Ala Val
       130                 135                 140
   gtg cat ccc ata gct tcc atg tct atc aga aca gag aag aac gca tta      480
   Val His Pro Ile Ala Ser Met Ser Ile Arg Thr Glu Lys Asn Ala Leu
   145                 150                 155                 160
   ctt gct ata gct tgc ata tgg gtg gtg ata tta acc aca gct att ccc      528
   Leu Ala Ile Ala Cys Ile Trp Val Val Ile Leu Thr Thr Ala Ile Pro
                   165                 170                 175
   gtg ggc atc tgc cac ggg gag cgg gaa tac tcc tac ttc cat aga aac      576
   Val Gly Ile Cys His Gly Glu Arg Glu Tyr Ser Tyr Phe His Arg Asn
               180                 185                 190
   cat tcc tcc tgc gtg ttc ctt gag gag caa gga tat agc aag cta ggg      624
   His Ser Ser Cys Val Phe Leu Glu Glu Gln Gly Tyr Ser Lys Leu Gly
           195                 200                 205
   ttc cag atg tct ttc ttt ttg tct tcc tat gtg att ccc tta gct ttg      672
   Phe Gln Met Ser Phe Phe Leu Ser Ser Tyr Val Ile Pro Leu Ala Leu
       210                 215                 220
   att agt gta ttg tat atg tgt atg ctg acg agg ttg tgg aag agt gct      720
   Ile Ser Val Leu Tyr Met Cys Met Leu Thr Arg Leu Trp Lys Ser Ala
   225                 230                 235                 240
   cca gga ggg agg gtg tct gcg gag agt cgg agg ggg agg aag aag gtg      768
   Pro Gly Gly Arg Val Ser Ala Glu Ser Arg Arg Gly Arg Lys Lys Val
                   245                 250                 255
   acg aga atg gtt gtg gtc gta gtg gtg gtg ttc gca gtg tgc tgg tgc      816
   Thr Arg Met Val Val Val Val Val Val Val Phe Ala Val Cys Trp Cys
               260                 265                 270
   cca ata cag ata atc ctg ttg gta aag gcg ctg gac aag tac cac atc      864
   Pro Ile Gln Ile Ile Leu Leu Val Lys Ala Leu Asp Lys Tyr His Ile
           275                 280                 285
   aca tac ttc aca gtc act gcg cag ata gtg tcg cat gta cta gcc tac      912
   Thr Tyr Phe Thr Val Thr Ala Gln Ile Val Ser His Val Leu Ala Tyr
       290                 295                 300
   atg aac agc tgc gtc aac cca gtg ctg tac gcc ttc ctc tct gag aac      960
   Met Asn Ser Cys Val Asn Pro Val Leu Tyr Ala Phe Leu Ser Glu Asn
   305                 310                 315                 320
   ttc agg gtc gcc ttc agg aag gta atg tac tgc ccg ccc ccc tac aat     1008
   Phe Arg Val Ala Phe Arg Lys Val Met Tyr Cys Pro Pro Pro Tyr Asn
                   325                 330                 335
   gac ggc atg tca ggc cgg ccc cag ccc acc aag acg act cgt aca ggc     1056
   Asp Gly Met Ser Gly Arg Pro Gln Pro Thr Lys Thr Thr Arg Thr Gly
               340                 345                 350
   aat ggg aac tcc tgc cat gac atc gtc tga                             1086
   Asn Gly Asn Ser Cys His Asp Ile Val
           355                 360
<210> 10
   <211> 361
   <212> PRT
   <213> spodoptera littoralis
<400> 10
   Met Ala Ser Thr Glu Asp Glu Phe Arg Ser Ile Cys Leu Asn Leu Thr
   1               5                   10                  15
   Ser Glu Glu Ala Met Leu Gly Gly Cys Asn Tyr Ser Ser Glu Tyr Glu
               20                  25                  30
   Asn Gly Glu Leu Leu Glu Lys Val Val Ser Arg Val Val Pro Ile Phe
           35                  40                  45
   Phe Gly Phe Ile Gly Ile Val Gly Leu Val Gly Asn Ala Leu Val Val
       50                  55                  60
   Leu Val Val Ala Ala Asn Pro Gly Met Arg Ser Thr Thr Asn Leu Leu
   65                  70                  75                  80
   Ile Ile Asn Leu Ala Val Ala Asp Leu Leu Phe Val Ile Phe Cys Val
                   85                  90                  95
   Pro Phe Thr Ala Thr Asp Tyr Val Met Pro Arg Trp Pro Phe Gly Asp
               100                 105                 110
   Trp Trp Cys Lys Val Val Gln Tyr Phe Ile Val Val Thr Ala His Ala
           115                 120                 125
   Ser Val Tyr Thr Leu Val Leu Met Ser Leu Asp Arg Phe Met Ala Val
       130                 135                 140
   Val His Pro Ile Ala Ser Met Ser Ile Arg Thr Glu Lys Asn Ala Leu
   145                 150                 155                 160
   Leu Ala Ile Ala Cys Ile Trp Val Val Ile Leu Thr Thr Ala Ile Pro
                   165                 170                 175
   Val Gly Ile Cys His Gly Glu Arg Glu Tyr Ser Tyr Phe His Arg Asn
               180                 185                 190
   His Ser Ser Cys Val Phe Leu Glu Glu Gln Gly Tyr Ser Lys Leu Gly
           195                 200                 205
   Phe Gln Met Ser Phe Phe Leu Ser Ser Tyr Val Ile Pro Leu Ala Leu
       210                 215                 220
   Ile Ser Val Leu Tyr Met Cys Met Leu Thr Arg Leu Trp Lys Ser Ala
   225                 230                 235                 240
   Pro Gly Gly Arg Val Ser Ala Glu Ser Arg Arg Gly Arg Lys Lys Val
                   245                 250                 255
   Thr Arg Met Val Val Val Val Val Val Val Phe Ala Val Cys Trp Cys
   260 265 270
   Pro Ile Gln Ile Ile Leu Leu Val Lys Ala Leu Asp Lys Tyr His Ile
   275 280 285
   Thr Tyr Phe Thr Val Thr Ala Gln Ile Val Ser His Val Leu Ala Tyr
   290 295 300
   Met Asn Ser Cys Val Asn Pro Val Leu Tyr Ala Phe Leu Ser Glu Asn
   305 310 315 320
   Phe Arg Val Ala Phe Arg Lys Val Met Tyr Cys Pro Pro Pro Tyr Asn
   325 330 335
   Asp Gly Met Ser Gly Arg Pro Gln Pro Thr Lys Thr Thr Arg Thr Gly
   340 345 350
   Asn Gly Asn Ser Cys His Asp Ile Val
   355 360
<210> 11
   <211> 1191
   <212> DNA
   <213> Spodoptera littoralis
<220>
   <221> CDS
   <222> (1)..(1191)
   <223> Diuretic hormone receptor encoding sequence
<400> 11
   atg gcg gag aag tgc ctg gcg cag aac gaa ggg ctg gcg gag gag gag       48
   Met Ala Glu Lys Cys Leu Ala Gln Asn Glu Gly Leu Ala Glu Glu Glu
   1               5                   10                  15
   ttc tgc ccc gcg tac tac gac ggg ctg ctg tgc tgg gac gcg acg ccc       96
   Phe Cys Pro Ala Tyr Tyr Asp Gly Leu Leu Cys Trp Asp Ala Thr Pro
               20                  25                  30
   tgg aac acg ctg gcg gtg cag cgc tgc ttc agc gag ttc cac ggc gtc      144
   Trp Asn Thr Leu Ala Val Gln Arg Cys Phe Ser Glu Phe His Gly Val
           35                  40                  45
   cag tac gat gac aca cag aac gca tcg cgg atg tgc ttg gac ggc gtg      192
   Gln Tyr Asp Asp Thr Gln Asn Ala Ser Arg Met Cys Leu Asp Gly Val
       50                  55                  60
   tgg cac aac tac tca aac tac gcg aac tgc aca gaa cgc atg acc aac      240
   Trp His Asn Tyr Ser Asn Tyr Ala Asn Cys Thr Glu Arg Met Thr Asn
   65                  70                  75                  80
   gtg tcg ccg act gac gtc acc agc ctc atc tac ctc gcc ggc tac tcg      288
   Val Ser Pro Thr Asp Val Thr Ser Leu Ile Tyr Leu Ala Gly Tyr Ser
                   85                  90                  95
   ctc agc ctc gcc gtg ctt tca ctc gcc gtc ttc gtc ttc ctt tat ttc      336
   Leu Ser Leu Ala Val Leu Ser Leu Ala Val Phe Val Phe Leu Tyr Phe
               100                 105                 110
   aag gac ctg cga tgt cta agg aac acg ata cat acc aac ctc atg tcg 384
   Lys Asp Leu Arg Cys Leu Arg Asn Thr Ile His Thr Asn Leu Met Ser
   115 120 125
   acg tat att ctg tct gcc tgc agc tgg atc tta aac tta gct tta caa 432
   Thr Tyr Ile Leu Ser Ala Cys Ser Trp Ile Leu Asn Leu Ala Leu Gln
   130 135 140
   aat tgg tcc gat gag gcc caa caa gat caa acc tcg tgt acg att ctg 480
   Asn Trp Ser Asp Glu Ala Gln Gln Asp Gln Thr Ser Cys Thr Ile Leu
   145 150 155 160
   gtc ata tgt atg cac tac ttt tac ctc act aat ttc ttc ggg atg ctt 528
   Val Ile Cys Met His Tyr Phe Tyr Leu Thr Asn Phe Phe Gly Met Leu
   165 170 175
   gta gaa ggt ttg tac ctg tac atg ctc gtg gtg gag acg ttc acc gct 576
   Val Glu Gly Leu Tyr Leu Tyr Met Leu Val Val Glu Thr Phe Thr Ala
   180 185 190
   gaa aat att aaa ctg aag gtg tac act acc ata gga tgg ggt gcg ccg 624
   Glu Asn Ile Lys Leu Lys Val Tyr Thr Thr Ile Gly Trp Gly Ala Pro
   195 200 205
   gcg gtg ttc cta aca ata tgg gtt ata tgc agg tgc ttc gtc aac atc 672
   Ala Val Phe Leu Thr Ile Trp Val Ile Cys Arg Cys Phe Val Asn Ile
   210 215 220
   gtg cct tcc aac cct gct gat ggg ctt gcc ctg gca ggt gag gct aag 720
   Val Pro Ser Asn Pro Ala Asp Gly Leu Ala Leu Ala Gly Glu Ala Lys
   225 230 235 240
   atg tgt att tgg atg tcg gag cac cag gtg gat tgg atc cat aaa gct 768
   Met Cys Ile Trp Met Ser Glu His Gln Val Asp Trp Ile His Lys Ala
   245 250 255
   cca gca ctc gct ggg ctc gcc ctc aac ctc ttc ttc ctc att agg att 816
   Pro Ala Leu Ala Gly Leu Ala Leu Asn Leu Phe Phe Leu Ile Arg Ile
   260 265 270
   atg tgg gta tta atc aca aaa ctg cgg tct gca aac acg ttg gag acg 864
   Met Trp Val Leu Ile Thr Lys Leu Arg Ser Ala Asn Thr Leu Glu Thr
   275 280 285
   gag cag tat cgg aaa gca acg aag gca ctt cta gtc ctg atc cca ctg 912
   Glu Gln Tyr Arg Lys Ala Thr Lys Ala Leu Leu Val Leu Ile Pro Leu
   290 295 300
   ctg ggc atc acc aac ctg ctg gtg ctg tgt ggg ccc aat gac gac tcc 960
   Leu Gly Ile Thr Asn Leu Leu Val Leu Cys Gly Pro Asn Asp Asp Ser
   305 310 315 320
   tgg ttc gca cac gcc ttc gac tac acg cga gca ctc atg ctt tca aca 1008
   Trp Phe Ala His Ala Phe Asp Tyr Thr Arg Ala Leu Met Leu Ser Thr
   325 330 335
   cag ggt ttc ttg gtg gct ttg ttc tac tgc ttc atg aat aca gaa gta 1056
   Gln Gly Phe Leu Val Ala Leu Phe Tyr Cys Phe Met Asn Thr Glu Val
   340 345 350
   cgg cac gca atc aga tac cac gtc gag agg tgg aag act ggg agg acc 1104
   Arg His Ala Ile Arg Tyr His Val Glu Arg Trp Lys Thr Gly Arg Thr
   355 360 365
   atc ggt gga gga agg agg aga gga gct tcc tac tcc aag gac tgg tct 1152
   Ile Gly Gly Gly Arg Arg Arg Gly Ala Ser Tyr Ser Lys Asp Trp Ser
   370 375 380
   cct cgg tca cga aca gaa agc ata cga ctc acg gta tga                 1191
   Pro Arg Ser Arg Thr Glu Ser Ile Arg Leu Thr Val
   385                 390                 395
<210> 12
   <211> 396
   <212> PRT
   <213> Spodoptera littoralis
<400> 12
   Met Ala Glu Lys Cys Leu Ala Gln Asn Glu Gly Leu Ala Glu Glu Glu
   1               5                   10                  15
   Phe Cys Pro Ala Tyr Tyr Asp Gly Leu Leu Cys Trp Asp Ala Thr Pro
               20                  25                  30
   Trp Asn Thr Leu Ala Val Gln Arg Cys Phe Ser Glu Phe His Gly Val
           35                  40                  45
   Gln Tyr Asp Asp Thr Gln Asn Ala Ser Arg Met Cys Leu Asp Gly Val
       50                  55                  60
   Trp His Asn Tyr Ser Asn Tyr Ala Asn Cys Thr Glu Arg Met Thr Asn
   65                  70                  75                  80
   Val Ser Pro Thr Asp Val Thr Ser Leu Ile Tyr Leu Ala Gly Tyr Ser
                   85                  90                  95
   Leu Ser Leu Ala Val Leu Ser Leu Ala Val Phe Val Phe Leu Tyr Phe
               100                 105                 110
   Lys Asp Leu Arg Cys Leu Arg Asn Thr Ile His Thr Asn Leu Met Ser
           115                 120                 125
   Thr Tyr Ile Leu Ser Ala Cys Ser Trp Ile Leu Asn Leu Ala Leu Gln
       130                 135                 140
   Asn Trp Ser Asp Glu Ala Gln Gln Asp Gln Thr Ser Cys Thr Ile Leu
   145                 150                 155                 160
   Val Ile Cys Met His Tyr Phe Tyr Leu Thr Asn Phe Phe Gly Met Leu
                   165                 170                 175
   Val Glu Gly Leu Tyr Leu Tyr Met Leu Val Val Glu Thr Phe Thr Ala
               180                 185                 190
   Glu Asn Ile Lys Leu Lys Val Tyr Thr Thr Ile Gly Trp Gly Ala Pro
           195                 200                 205
   Ala Val Phe Leu Thr Ile Trp Val Ile Cys Arg Cys Phe Val Asn Ile
       210                 215                 220
   Val Pro Ser Asn Pro Ala Asp Gly Leu Ala Leu Ala Gly Glu Ala Lys
   225                 230                 235                 240
   Met Cys Ile Trp Met Ser Glu His Gln Val Asp Trp Ile His Lys Ala
                   245                 250                 255
   Pro Ala Leu Ala Gly Leu Ala Leu Asn Leu Phe Phe Leu Ile Arg Ile
               260                 265                 270
   Met Trp Val Leu Ile Thr Lys Leu Arg Ser Ala Asn Thr Leu Glu Thr
           275                 280                 285
   Glu Gln Tyr Arg Lys Ala Thr Lys Ala Leu Leu Val Leu Ile Pro Leu
       290                 295                 300
   Leu Gly Ile Thr Asn Leu Leu Val Leu Cys Gly Pro Asn Asp Asp Ser
   305                 310                 315                 320
   Trp Phe Ala His Ala Phe Asp Tyr Thr Arg Ala Leu Met Leu Ser Thr
                   325                 330                 335
   Gln Gly Phe Leu Val Ala Leu Phe Tyr Cys Phe Met Asn Thr Glu Val
               340                 345                 350
   Arg His Ala Ile Arg Tyr His Val Glu Arg Trp Lys Thr Gly Arg Thr
           355                 360                 365
   Ile Gly Gly Gly Arg Arg Arg Gly Ala Ser Tyr Ser Lys Asp Trp Ser
       370                 375                 380
   Pro Arg Ser Arg Thr Glu Ser Ile Arg Leu Thr Val
   385                 390                 395
<210> 13
   <211> 2250
   <212> DNA
   <213> Spodoptera littoralis
<220>
   <221> CDS
   <222> (1)..(2250)
   <223> LGR1 receptor encoding sequence
<400> 13
   atg tat tgg aga tta tgt att tgg gct gtt gcc ttt att tta ctg ggg 48
   Met Tyr Trp Arg Leu Cys Ile Trp Ala Val Ala Phe Ile Leu Leu Gly
   1 5 10 15
   agc aga gaa gca gca tgc cac tcg atg agt gag agt gtg tct gtg gtg 96
   Ser Arg Glu Ala Ala Cys His Ser Met Ser Glu Ser Val Ser Val Val
   20 25 30
   ctg cac cag acc aac atc aca cgg ctc acg gca aag gtc atc cat gct 144
   Leu His Gln Thr Asn Ile Thr Arg Leu Thr Ala Lys Val Ile His Ala
   35 40 45
   gcg ggc tac cat ttg cat cga gtt gag cat tta tca ata atg gat gca 192
   Ala Gly Tyr His Leu His Arg Val Glu His Leu Ser Ile Met Asp Ala
   50 55 60
   cca aaa ttg gat cac ata gta gtg gac gat ctg act aag atg cct aat 240
   Pro Lys Leu Asp His Ile Val Val Asp Asp Leu Thr Lys Met Pro Asn
   65 70 75 80
   ctc aaa tct ctg ttt ata acg caa gct ccg cga cta cac agg gtg tcg 288
   Leu Lys Ser Leu Phe Ile Thr Gln Ala Pro Arg Leu His Arg Val Ser
   85 90 95
   ccc ctc cct ccg ctg ccc gag cta cgc aca ttc atc atc aca acc tcc 336
   Pro Leu Pro Pro Leu Pro Glu Leu Arg Thr Phe Ile Ile Thr Thr Ser
   100 105 110
   ggc ttg gtg gaa gtc ccc aac ctg agc cac gtg cac gac aag aag aat 384
   Gly Leu Val Glu Val Pro Asn Leu Ser His Val His Asp Lys Lys Asn
   115 120 125
   aac aac tca ctc gcc tat ctg caa gct ata gac cta gaa ggc aac cac 432
   Asn Asn Ser Leu Ala Tyr Leu Gln Ala Ile Asp Leu Glu Gly Asn His
   130 135 140
   ata aag cgc cta tcg ccg cac gca ttg cgc gtg cgc gct gat caa gtg 480
   Ile Lys Arg Leu Ser Pro His Ala Leu Arg Val Arg Ala Asp Gln Val
   145 150 155 160
   tcc ttg aac tac aac ttg cta gaa gaa gtg cct gct ttt gcg ttc aag 528
   Ser Leu Asn Tyr Asn Leu Leu Glu Glu Val Pro Ala Phe Ala Phe Lys
   165 170 175
   aat gcg cag att tca aaa ttg agt ttc aga gga aac acg aaa cta aaa 576
   Asn Ala Gln Ile Ser Lys Leu Ser Phe Arg Gly Asn Thr Lys Leu Lys
   180 185 190
   cat att gat gaa aat gcc ttc gca tcg aat ttg ctc ctc cga caa tta 624
   His Ile Asp Glu Asn Ala Phe Ala Ser Asn Leu Leu Leu Arg Gln Leu
   195 200 205
   gat tta agt aac acc gct att acc tcc ctg cct aca gca gga cta cag 672
   Asp Leu Ser Asn Thr Ala Ile Thr Ser Leu Pro Thr Ala Gly Leu Gln
   210 215 220
   aac ttg gaa acc ctt aga ata gaa aag aca cct tca ttg aaa tac atc 720
   Asn Leu Glu Thr Leu Arg Ile Glu Lys Thr Pro Ser Leu Lys Tyr Ile
   225 230 235 240
   cca tcg att tac gaa ttt cag agt ctc cag aaa gcg tac ctg acg cat 768
   Pro Ser Ile Tyr Glu Phe Gln Ser Leu Gln Lys Ala Tyr Leu Thr His
   245 250 255
   cat ttc cac tgc tgc gcg ttc cac ttc ccc gag cga cac aac cca acg 816
   His Phe His Cys Cys Ala Phe His Phe Pro Glu Arg His Asn Pro Thr
   260 265 270
   aga cac aag ctc tat gaa acg caa att gct ata atg cag tcg aaa tgt 864
   Arg His Lys Leu Tyr Glu Thr Gln Ile Ala Ile Met Gln Ser Lys Cys
   275 280 285
   gcg agc gct agt cag cag aag aaa cca gta gcc aga aga aag aag agg 912
   Ala Ser Ala Ser Gln Gln Lys Lys Pro Val Ala Arg Arg Lys Lys Arg
   290 295 300
   tca ctg gca cca ctc aag atc gtt atg cct cag cgt aaa aac gat act 960
   Ser Leu Ala Pro Leu Lys Ile Val Met Pro Gln Arg Lys Asn Asp Thr
   305 310 315 320
   aca gac tca tta gag gaa gac ctg tca aca gcg act acg agc gag gag 1008
   Thr Asp Ser Leu Glu Glu Asp Leu Ser Thr Ala Thr Thr Ser Glu Glu
   325 330 335
   tac gac gct aac ttg atg gac gag tac ttc tca gac tcc ggc agt tgg 1056
   Tyr Asp Ala Asn Leu Met Asp Glu Tyr Phe Ser Asp Ser Gly Ser Trp
   340 345 350
   gag gac gag gac gac cag gga gag ttc cac gaa cca gtc aac aac acg 1104
   Glu Asp Glu Asp Asp Gln Gly Glu Phe His Glu Pro Val Asn Asn Thr
   355 360 365
   ctc ggg gcc ggc ttc gcg atc ata tcc gct gag tgc ggg aac ttc agt 1152
   Leu Gly Ala Gly Phe Ala Ile Ile Ser Ala Glu Cys Gly Asn Phe Ser
   370 375 380
   att agc cca cga aaa gtg gaa tgc ttt ccc gca cct gat gca ctg aat 1200
   Ile Ser Pro Arg Lys Val Glu Cys Phe Pro Ala Pro Asp Ala Leu Asn
   385 390 395 400
   ccg tgc gaa gat gtg atg ggc tgg tcg tgg ctc cga gcg agt gtg tgg 1248
   Pro Cys Glu Asp Val Met Gly Trp Ser Trp Leu Arg Ala Ser Val Trp
   405 410 415
   ttc gtc atc acg gcg gcg gtg gtg ggc aac gtt gcc gtc ctg ctc gtc 1296
   Phe Val Ile Thr Ala Ala Val Val Gly Asn Val Ala Val Leu Leu Val
   420 425 430
   tta ctt aca aac cac acg gag att aca gtc ccg cgg ttc ctt atg tgc 1344
   Leu Leu Thr Asn His Thr Glu Ile Thr Val Pro Arg Phe Leu Met Cys
   435 440 445
   cac ctc gcc ttc tct gac ctc tgc acc ggc ctc tac ctg ttt atg ttg 1392
   His Leu Ala Phe Ser Asp Leu Cys Thr Gly Leu Tyr Leu Phe Met Leu
   450 455 460
   gcc gtc gta gac ctc aga tct tat gga gaa ttc ttc aac tac gcc tac 1440
   Ala Val Val Asp Leu Arg Ser Tyr Gly Glu Phe Phe Asn Tyr Ala Tyr
   465 470 475 480
   gat tgg cag tac ggg gtc ggc tgc aag atc gct ggc ttt cta tca gta 1488
   Asp Trp Gln Tyr Gly Val Gly Cys Lys Ile Ala Gly Phe Leu Ser Val
   485 490 495
   ttt tct gga cag tta tca gta att act tta act ata gtc aca ttg gag 1536
   Phe Ser Gly Gln Leu Ser Val Ile Thr Leu Thr Ile Val Thr Leu Glu
   500 505 510
   cgc tgg ttc gcc ata act tat gca ata tac tta gaa agg agg atc tct 1584
   Arg Trp Phe Ala Ile Thr Tyr Ala Ile Tyr Leu Glu Arg Arg Ile Ser
   515 520 525
   cta tca gcc gca gct aag ata atg atg ggc ggt tgg ttg tac tcc ttc 1632
   Leu Ser Ala Ala Ala Lys Ile Met Met Gly Gly Trp Leu Tyr Ser Phe
   530 535 540
   ctg atg gct ggc ttg ccg ctg gtc ggc gtg tca gac tat tcg tct acg 1680
   Leu Met Ala Gly Leu Pro Leu Val Gly Val Ser Asp Tyr Ser Ser Thr
   545 550 555 560
   tca ata tgt tta ccg gtt gag agc aag gat gtg gga gat gct gtg tac 1728
   Ser Ile Cys Leu Pro Val Glu Ser Lys Asp Val Gly Asp Ala Val Tyr
   565 570 575
   cag gga tca ata ttt ata gtg agt gcg ttg gcg tgg gtc acg att gtg      1776
   Gln Gly Ser Ile Phe Ile Val Ser Ala Leu Ala Trp Val Thr Ile Val
               580                 585                 590
   gtg tgc tac gtg cag ata tac agg tcg cta ggg ggt gga gag agt tac      1824
   Val Cys Tyr Val Gln Ile Tyr Arg Ser Leu Gly Gly Gly Glu Ser Tyr
           595                 600                 605
   gga ggg agg ggg gcg gcc gca gcc gct gag aga cga att gcg aat aaa      1872
   Gly Gly Arg Gly Ala Ala Ala Ala Ala Glu Arg Arg Ile Ala Asn Lys
       610                 615                 620
   atg gca ctc ctc atc gga acc gac ctg ttg tgc tgg gcg cct gtg gcg      1920
   Met Ala Leu Leu Ile Gly Thr Asp Leu Leu Cys Trp Ala Pro Val Ala
   625                 630                 635                 640
   ttc ttc ggc atc aca gct cta gca gga gtg ccg cta gtc gat gtc agt      1968
   Phe Phe Gly Ile Thr Ala Leu Ala Gly Val Pro Leu Val Asp Val Ser
                   645                 650                 655
   cac ggg aaa gtg ttg cta gta ttc ttt tac cct ctg aac gct tgt gcg      2016
   His Gly Lys Val Leu Leu Val Phe Phe Tyr Pro Leu Asn Ala Cys Ala
               660                 665                 670
   aat cca ttc tta tac gcc ata tta acg aaa caa tac cga aga gat ttg      2064
   Asn Pro Phe Leu Tyr Ala Ile Leu Thr Lys Gln Tyr Arg Arg Asp Leu
           675                 680                 685
   ata acg ttg ata gca cgg acg ggt cac tgc acc tgg tta gtg gag aag      2112
   Ile Thr Leu Ile Ala Arg Thr Gly His Cys Thr Trp Leu Val Glu Lys
       690                 695                 700
   tac aag ttg gcg gcg acc ccg ccc ccc acc gcg cac act aac cct tct      2160
   Tyr Lys Leu Ala Ala Thr Pro Pro Pro Thr Ala His Thr Asn Pro Ser
   705                 710                 715                 720
   gcg ccg gcg cag ttg atg ccg cta gta gac tcc gtc acg tat caa agg      2208
   Ala Pro Ala Gln Leu Met Pro Leu Val Asp Ser Val Thr Tyr Gln Arg
                   725                 730                 735
   agc cag tca cag tgt aaa gac agt agt gag gta ccg ctt taa              2250
   Ser Gln Ser Gln Cys Lys Asp Ser Ser Glu Val Pro Leu
               740                 745
<210> 14
   <211> 749
   <212> PRT
   <213> spodoptera littoralis
<400> 14
   Met Tyr Trp Arg Leu Cys Ile Trp Ala Val Ala Phe Ile Leu Leu Gly
   1               5                   10                  15
   Ser Arg Glu Ala Ala Cys His Ser Met Ser Glu Ser Val Ser Val Val
               20                  25                  30
   Leu His Gln Thr Asn Ile Thr Arg Leu Thr Ala Lys Val Ile His Ala
           35                  40                  45
   Ala Gly Tyr His Leu His Arg Val Glu His Leu Ser Ile Met Asp Ala
       50                  55                  60
   Pro Lys Leu Asp His Ile Val Val Asp Asp Leu Thr Lys Met Pro Asn
   65                  70                  75                  80
   Leu Lys Ser Leu Phe Ile Thr Gln Ala Pro Arg Leu His Arg Val Ser
                   85                  90                  95
   Pro Leu Pro Pro Leu Pro Glu Leu Arg Thr Phe Ile Ile Thr Thr Ser
               100                 105                 110
   Gly Leu Val Glu Val Pro Asn Leu Ser His Val His Asp Lys Lys Asn
           115                 120                 125
   Asn Asn Ser Leu Ala Tyr Leu Gln Ala Ile Asp Leu Glu Gly Asn His
       130                 135                 140
   Ile Lys Arg Leu Ser Pro His Ala Leu Arg Val Arg Ala Asp Gln Val
   145                 150                 155                 160
   Ser Leu Asn Tyr Asn Leu Leu Glu Glu Val Pro Ala Phe Ala Phe Lys
                   165                 170                 175
   Asn Ala Gln Ile Ser Lys Leu Ser Phe Arg Gly Asn Thr Lys Leu Lys
               180                 185                 190
   His Ile Asp Glu Asn Ala Phe Ala Ser Asn Leu Leu Leu Arg Gln Leu
           195                 200                 205
   Asp Leu Ser Asn Thr Ala Ile Thr Ser Leu Pro Thr Ala Gly Leu Gln
       210                 215                 220
   Asn Leu Glu Thr Leu Arg Ile Glu Lys Thr Pro Ser Leu Lys Tyr Ile
   225                 230                 235                 240
   Pro Ser Ile Tyr Glu Phe Gln Ser Leu Gln Lys Ala Tyr Leu Thr His
                   245                 250                 255
   His Phe His Cys Cys Ala Phe His Phe Pro Glu Arg His Asn Pro Thr
               260                 265                 270
   Arg His Lys Leu Tyr Glu Thr Gln Ile Ala Ile Met Gln Ser Lys Cys
           275                 280                 285
   Ala Ser Ala Ser Gln Gln Lys Lys Pro Val Ala Arg Arg Lys Lys Arg
       290                 295                 300
   Ser Leu Ala Pro Leu Lys Ile Val Met Pro Gln Arg Lys Asn Asp Thr
   305                 310                 315                 320
   Thr Asp Ser Leu Glu Glu Asp Leu Ser Thr Ala Thr Thr Ser Glu Glu
                   325                 330                 335
   Tyr Asp Ala Asn Leu Met Asp Glu Tyr Phe Ser Asp Ser Gly Ser Trp
               340                 345                 350
   Glu Asp Glu Asp Asp Gln Gly Glu Phe His Glu Pro Val Asn Asn Thr
           355                     360                 365
   Leu Gly Ala Gly Phe Ala Ile Ile Ser Ala Glu Cys Gly Asn Phe Ser
       370                     375                 380
   Ile Ser Pro Arg Lys Val Glu Cys Phe Pro Ala Pro Asp Ala Leu Asn
   385                 390                 395                     400
   Pro Cys Glu Asp Val Met Gly Trp Ser Trp Leu Arg Ala Ser Val Trp
                   405                 410                     415
   Phe Val Ile Thr Ala Ala Val Val Gly Asn Val Ala Val Leu Leu Val
               420                 425                 430
   Leu Leu Thr Asn His Thr Glu Ile Thr Val Pro Arg Phe Leu Met Cys
           435                     440                 445
   His Leu Ala Phe Ser Asp Leu Cys Thr Gly Leu Tyr Leu Phe Met Leu
       450                     455                 460
   Ala Val Val Asp Leu Arg Ser Tyr Gly Glu Phe Phe Asn Tyr Ala Tyr
   465                 470                 475                     480
   Asp Trp Gln Tyr Gly Val Gly Cys Lys Ile Ala Gly Phe Leu Ser Val
                   485                 490                     495
   Phe Ser Gly Gln Leu Ser Val Ile Thr Leu Thr Ile Val Thr Leu Glu
               500                 505                 510
   Arg Trp Phe Ala Ile Thr Tyr Ala Ile Tyr Leu Glu Arg Arg Ile Ser
           515                     520                 525
   Leu Ser Ala Ala Ala Lys Ile Met Met Gly Gly Trp Leu Tyr Ser Phe
       530                     535                 540
   Leu Met Ala Gly Leu Pro Leu Val Gly Val Ser Asp Tyr Ser Ser Thr
   545                 550                 555                     560
   Ser Ile Cys Leu Pro Val Glu Ser Lys Asp Val Gly Asp Ala Val Tyr
                   565                 570                     575
   Gln Gly Ser Ile Phe Ile Val Ser Ala Leu Ala Trp Val Thr Ile Val
               580                 585                 590
   Val Cys Tyr Val Gln Ile Tyr Arg Ser Leu Gly Gly Gly Glu Ser Tyr
           595                     600                 605
   Gly Gly Arg Gly Ala Ala Ala Ala Ala Glu Arg Arg Ile Ala Asn Lys
       610                     615                 620
   Met Ala Leu Leu Ile Gly Thr Asp Leu Leu Cys Trp Ala Pro Val Ala
   625 630 635 640
   Phe Phe Gly Ile Thr Ala Leu Ala Gly Val Pro Leu Val Asp Val Ser
   645 650 655
   His Gly Lys Val Leu Leu Val Phe Phe Tyr Pro Leu Asn Ala Cys Ala
   660 665 670
   Asn Pro Phe Leu Tyr Ala Ile Leu Thr Lys Gln Tyr Arg Arg Asp Leu
   675 680 685
   Ile Thr Leu Ile Ala Arg Thr Gly His Cys Thr Trp Leu Val Glu Lys
   690 695 700
   Tyr Lys Leu Ala Ala Thr Pro Pro Pro Thr Ala His Thr Asn Pro Ser
   705 710 715 720
   Ala Pro Ala Gln Leu Met Pro Leu Val Asp Ser Val Thr Tyr Gln Arg
   725 730 735
   Ser Gln Ser Gln Cys Lys Asp Ser Ser Glu Val Pro Leu
   740 745
<210> 15
   <211> 1437
   <212> DNA
   <213> Spodoptera littoralis
<220>
   <221> CDS
   <222> (1)..(1437)
   <223> OCt/Tyr receptor encoding sequence
<400> 15
   atg ggg caa aca gct aca cac gta aac gca aat tac aca tta att aat      48
   Met Gly Gln Thr Ala Thr His Val Asn Ala Asn Tyr Thr Leu Ile Asn
   1               5                   10                  15
   tat act gat gaa ata att gag gat gac aga gac gct tgt gcc gtc gca      96
   Tyr Thr Asp Glu Ile Ile Glu Asp Asp Arg Asp Ala Cys Ala Val Ala
               20                  25                  30
   gat gac cct aaa tat ccg agt agt ttc ggt ata aca ctt gcg gta cca      144
   Asp Asp Pro Lys Tyr Pro Ser Ser Phe Gly Ile Thr Leu Ala Val Pro
           35                  40                  45
   gaa tgg gaa gca att tgt aca gcc ata gtc cta acg ctt ata ata atc      192
   Glu Trp Glu Ala Ile Cys Thr Ala Ile Val Leu Thr Leu Ile Ile Ile
       50                  55                  60
   tca acg ata gtt gga aac att ttg gtg atc ttg agt gtg ttt aca tac      240
   Ser Thr Ile Val Gly Asn Ile Leu Val Ile Leu Ser Val Phe Thr Tyr
   65                  70                  75                  80
   aaa ccc ctt agg att gtc cag aac ttc ttc ata gta tca ctt gct gtg      288
   Lys Pro Leu Arg Ile Val Gln Asn Phe Phe Ile Val Ser Leu Ala Val
                   85                  90                  95
   gcg gat ttg aca gta gca ata tta gta ttg cca cta aat gtg gca tac      336
   Ala Asp Leu Thr Val Ala Ile Leu Val Leu Pro Leu Asn Val Ala Tyr
               100                 105                 110
   tcc att cta gga caa tgg gta ttt ggg att tac gta tgc aaa atg tgg      384
   Ser Ile Leu Gly Gln Trp Val Phe Gly Ile Tyr Val Cys Lys Met Trp
           115                 120                 125
   tta aca tgc gac ata atg tgt tgc act tcg tcc att tta aat tta tgt      432
   Leu Thr Cys Asp Ile Met Cys Cys Thr Ser Ser Ile Leu Asn Leu Cys
       130                 135                 140
   gca atc gca tta gac agg tat tgg gcg ata acc gat cct ata aat tat      480
   Ala Ile Ala Leu Asp Arg Tyr Trp Ala Ile Thr Asp Pro Ile Asn Tyr
   145                 150                 155                 160
   gca caa aag aga act tta gaa aga gtg atg tta atg att gga gta gtt      528
   Ala Gln Lys Arg Thr Leu Glu Arg Val Met Leu Met Ile Gly Val Val
                   165                 170                 175
   tgg gta ctt tca tta gtt att agc tct ccg cca ctt cta ggg tgg aat      576
   Trp Val Leu Ser Leu Val Ile Ser Ser Pro Pro Leu Leu Gly Trp Asn
               180                 185                 190
   gac tgg ccc gac gtc ttt gaa cct gat acg cct tgt cgt tta act tcc      624
   Asp Trp Pro Asp Val Phe Glu Pro Asp Thr Pro Cys Arg Leu Thr Ser
           195                 200                 205
   caa cct ggc ttc gtt atc ttc tct tca tct ggg tcc ttc tac ata ccg      672
   Gln Pro Gly Phe Val Ile Phe Ser Ser Ser Gly Ser Phe Tyr Ile Pro
       210                 215                 220
   cta gtt ata atg act gta gtt tat ttt gaa ata tat ttg gcc act aaa      720
   Leu Val Ile Met Thr Val Val Tyr Phe Glu Ile Tyr Leu Ala Thr Lys
   225                 230                 235                 240
   aaa aga ctt aga gat cgc gct aaa gcc aca aag atc agc act att tct      768
   Lys Arg Leu Arg Asp Arg Ala Lys Ala Thr Lys Ile Ser Thr Ile Ser
                   245                 250                 255
   agc ggt cag aat aga tac aat aat aaa gac gat aac aat gat cat gat      816
   Ser Gly Gln Asn Arg Tyr Asn Asn Lys Asp Asp Asn Asn Asp His Asp
               260                 265                 270
   caa gat tct gtt agc tca gaa gcc aat cac aat gag cac caa ggc gtg      864
   Gln Asp Ser Val Ser Ser Glu Ala Asn His Asn Glu His Gln Gly Val
           275                 280                 285
   aca cgg ttg gca tca gac aat gag aaa aag aaa aga acc aga aaa ttg      912
   Thr Arg Leu Ala Ser Asp Asn Glu Lys Lys Lys Arg Thr Arg Lys Leu
       290                 295                 300
   aca cca aag aaa aaa cca aag cga cga tat tgg agt aag gac gat aaa      960
   Thr Pro Lys Lys Lys Pro Lys Arg Arg Tyr Trp Ser Lys Asp Asp Lys
   305                 310                 315                 320
   tcg caa aac aaa ctg ata ata ccg att ctc tct aac gat aat tcg gta     1008
   Ser Gln Asn Lys Leu Ile Ile Pro Ile Leu Ser Asn Asp Asn Ser Val
                   325                 330                 335
   act gat atg ggt gat aat tta gaa aat aga aac aca tca tca gag agt     1056
   Thr Asp Met Gly Asp Asn Leu Glu Asn Arg Asn Thr Ser Ser Glu Ser
               340                 345                 350
   aac tcg aaa gaa act cac gag gat gat ttg att gaa gtt act gag cct     1104
   Asn Ser Lys Glu Thr His Glu Asp Asp Leu Ile Glu Val Thr Glu Pro
           355                 360                 365
   gcg cca gtg aag agt cac aag cgg ccg aag cct aac cag caa agt gct      1152
   Ala Pro Val Lys Ser His Lys Arg Pro Lys Pro Asn Gln Gln Ser Ala
       370                 375                 380
   gtt tat caa ttc att gaa gaa aag caa agg att tcc ttg aca cgt gag      1200
   Val Tyr Gln Phe Ile Glu Glu Lys Gln Arg Ile Ser Leu Thr Arg Glu
   385                 390                 395                 400
   cgt cga gcc gcc aga acc ctt ggg att ata atg ggc gta ttt gtc gtg      1248
   Arg Arg Ala Ala Arg Thr Leu Gly Ile Ile Met Gly Val Phe Val Val
                   405                 410                 415
   tgt tgg ttg ccc ttc ttt gtc ata tac ctt gtt att ccc ttc tgt gcg      1296
   Cys Trp Leu Pro Phe Phe Val Ile Tyr Leu Val Ile Pro Phe Cys Ala
               420                 425                 430
   agt tgc tgt ttg tcc aac aag ttt atc aat ttc ata act tgg ctt ggc      1344
   Ser Cys Cys Leu Ser Asn Lys Phe Ile Asn Phe Ile Thr Trp Leu Gly
           435                 440                 445
   tac tgc aat tct gca ctc aac cct cta ata tac acc atc ttt aac atg      1392
   Tyr Cys Asn Ser Ala Leu Asn Pro Leu Ile Tyr Thr Ile Phe Asn Met
       450                 455                 460
   gac ttt agg aga gca ttc aaa aag tta ctc tgt atg aaa ccg tga          1437
   Asp Phe Arg Arg Ala Phe Lys Lys Leu Leu Cys Met Lys Pro
   465                 470                 475
<210> 16
   <211> 478
   <212> PRT
   <213> Spodoptera littoralis
<400> 16
   Met Gly Gln Thr Ala Thr His Val Asn Ala Asn Tyr Thr Leu Ile Asn
   1               5                   10                  15
   Tyr Thr Asp Glu Ile Ile Glu Asp Asp Arg Asp Ala Cys Ala Val Ala
               20                  25                  30
   Asp Asp Pro Lys Tyr Pro Ser Ser Phe Gly Ile Thr Leu Ala Val Pro
           35                  40                  45
   Glu Trp Glu Ala Ile Cys Thr Ala Ile Val Leu Thr Leu Ile Ile Ile
       50                  55                  60
   Ser Thr Ile Val Gly Asn Ile Leu Val Ile Leu Ser Val Phe Thr Tyr
   65                  70                  75                  80
   Lys Pro Leu Arg Ile Val Gln Asn Phe Phe Ile Val Ser Leu Ala Val
                   85                  90                  95
   Ala Asp Leu Thr Val Ala Ile Leu Val Leu Pro Leu Asn Val Ala Tyr
               100                 105                 110
   Ser Ile Leu Gly Gln Trp Val Phe Gly Ile Tyr Val Cys Lys Met Trp
           115                 120                 125
   Leu Thr Cys Asp Ile Met Cys Cys Thr Ser Ser Ile Leu Asn Leu Cys
       130                 135                 140
   Ala Ile Ala Leu Asp Arg Tyr Trp Ala Ile Thr Asp Pro Ile Asn Tyr
   145                 150                 155                 160
   Ala Gln Lys Arg Thr Leu Glu Arg Val Met Leu Met Ile Gly Val Val
                   165                 170                 175
   Trp Val Leu Ser Leu Val Ile Ser Ser Pro Pro Leu Leu Gly Trp Asn
               180                 185                 190
   Asp Trp Pro Asp Val Phe Glu Pro Asp Thr Pro Cys Arg Leu Thr Ser
           195                 200                 205
   Gln Pro Gly Phe Val Ile Phe Ser Ser Ser Gly Ser Phe Tyr Ile Pro
       210                 215                 220
   Leu Val Ile Met Thr Val Val Tyr Phe Glu Ile Tyr Leu Ala Thr Lys
   225                 230                 235                 240
   Lys Arg Leu Arg Asp Arg Ala Lys Ala Thr Lys Ile Ser Thr Ile Ser
                   245                 250                 255
   Ser Gly Gln Asn Arg Tyr Asn Asn Lys Asp Asp Asn Asn Asp His Asp
               260                 265                 270
   Gln Asp Ser Val Ser Ser Glu Ala Asn His Asn Glu His Gln Gly Val
           275                 280                 285
   Thr Arg Leu Ala Ser Asp Asn Glu Lys Lys Lys Arg Thr Arg Lys Leu
       290                 295                 300
   Thr Pro Lys Lys Lys Pro Lys Arg Arg Tyr Trp Ser Lys Asp Asp Lys
   305                 310                 315                 320
   Ser Gln Asn Lys Leu Ile Ile Pro Ile Leu Ser Asn Asp Asn Ser Val
                   325                 330                 335
   Thr Asp Met Gly Asp Asn Leu Glu Asn Arg Asn Thr Ser Ser Glu Ser
               340                 345                 350
   Asn Ser Lys Glu Thr His Glu Asp Asp Leu Ile Glu Val Thr Glu Pro
           355                 360                 365
   Ala Pro Val Lys Ser His Lys Arg Pro Lys Pro Asn Gln Gln Ser Ala
       370                 375                 380
   Val Tyr Gln Phe Ile Glu Glu Lys Gln Arg Ile Ser Leu Thr Arg Glu
   385                 390                 395                 400
   Arg Arg Ala Ala Arg Thr Leu Gly Ile Ile Met Gly Val Phe Val Val
                   405                 410                 415
   Cys Trp Leu Pro Phe Phe Val Ile Tyr Leu Val Ile Pro Phe Cys Ala
               420                 425                 430
   Ser Cys Cys Leu Ser Asn Lys Phe Ile Asn Phe Ile Thr Trp Leu Gly
           435                 440                 445
   Tyr Cys Asn Ser Ala Leu Asn Pro Leu Ile Tyr Thr Ile Phe Asn Met
       450                 455                 460
   Asp Phe Arg Arg Ala Phe Lys Lys Leu Leu Cys Met Lys Pro
   465                 470                 475
<210> 17
   <211> 1419
   <212> DNA
   <213> Spodoptera littoralis
<220>
   <221> CDS
   <222> (1)..(1419)
   <223> G protein coupled receptor SlOR83b encoding sequence
<400> 17
   atg acc aaa gtg aag gcc cag ggc ctc gtc tcc gac ctg atg ccc aac      48
   Met Thr Lys Val Lys Ala Gln Gly Leu Val Ser Asp Leu Met Pro Asn
   1               5                   10                  15
   atc aag ttg atg cag gca gct gga cac ttc ctc ttc aat tac cat gca      96
   Ile Lys Leu Met Gln Ala Ala Gly His Phe Leu Phe Asn Tyr His Ala
               20                  25                  30
   gaa aat ggt ggc atg agc ggc ctc ctt cgg aag atc tac gcc agt acc     144
   Glu Asn Gly Gly Met Ser Gly Leu Leu Arg Lys Ile Tyr Ala Ser Thr
           35                  40                  45
   cac gcg att ctc atc acc ata cat ttt gcc tgt atg ggt atc aac atg     192
   His Ala Ile Leu Ile Thr Ile His Phe Ala Cys Met Gly Ile Asn Met
       50                  55                  60
   gcc cag tac tcc gat gag gtc aac gag ctg act gct aac act atc acc     240
   Ala Gln Tyr Ser Asp Glu Val Asn Glu Leu Thr Ala Asn Thr Ile Thr
   65                  70                  75                  80
   gtc ctc ttc ttc act cac acc atc atc aag ttg ggg ttc ttc gct ttg     288
   Val Leu Phe Phe Thr His Thr Ile Ile Lys Leu Gly Phe Phe Ala Leu
                   85                  90                  95
   aac tcc aag agt ttt tac agg act tta gca gta tgg aac cag tcg aac     336
   Asn Ser Lys Ser Phe Tyr Arg Thr Leu Ala Val Trp Asn Gln Ser Asn
               100                 105                 110
   agc cac cct ctg ttc acg gag tca gat gcc cgc tac cat caa att gcg     384
   Ser His Pro Leu Phe Thr Glu Ser Asp Ala Arg Tyr His Gln Ile Ala
           115                 120                 125
   ctc acc aag atg agg aga ctc ctg tac ttc atc tgc ggc atg act gtc     432
   Leu Thr Lys Met Arg Arg Leu Leu Tyr Phe Ile Cys Gly Met Thr Val
       130                 135                 140
   ctg tca gtt gtt agc tgg gtc aca ctc aca ttc ttc ggt gaa tca gtc 480
   Leu Ser Val Val Ser Trp Val Thr Leu Thr Phe Phe Gly Glu Ser Val
   145 150 155 160
   cgc ctg att act agt aag gaa acg aat gag acg ttg aca gaa gta gcc 528
   Arg Leu Ile Thr Ser Lys Glu Thr Asn Glu Thr Leu Thr Glu Val Ala
   165 170 175
   cct cga ttg cct ctg aag gcc tgg tac cct ttc aat gct atg agc ggc 576
   Pro Arg Leu Pro Leu Lys Ala Trp Tyr Pro Phe Asn Ala Met Ser Gly
   180 185 190
   acc acg tat att atc gct ttc gct ttc cag gtc tac tgg ctc ctc ttc 624
   Thr Thr Tyr Ile Ile Ala Phe Ala Phe Gln Val Tyr Trp Leu Leu Phe
   195 200 205
   tca atg gct ata gca aat ctg atg gat gtt atg ttc tgc tcc tgg ctg 672
   Ser Met Ala Ile Ala Asn Leu Met Asp Val Met Phe Cys Ser Trp Leu
   210 215 220
   atc ttc gct tgt gaa cag ctg cag cac ctg aag gcc atc atg aaa cct 720
   Ile Phe Ala Cys Glu Gln Leu Gln His Leu Lys Ala Ile Met Lys Pro
   225 230 235 240
   ctc atg gaa ctc agt gct tca ctg gac acg tat cgg cct aat act gct 768
   Leu Met Glu Leu Ser Ala Ser Leu Asp Thr Tyr Arg Pro Asn Thr Ala
   245 250 255
   gag ctg ttc cgt gct tct tcc act gag aaa tca gaa aag att cct gat 816
   Glu Leu Phe Arg Ala Ser Ser Thr Glu Lys Ser Glu Lys Ile Pro Asp
   260 265 270
   aca gtc gac atg gac atc cgc ggc ata tat tcc aca cag caa gat ttc 864
   Thr Val Asp Met Asp Ile Arg Gly Ile Tyr Ser Thr Gln Gln Asp Phe
   275 280 285
   ggg atg acg ttg cga gga gct gga ggt aga ctg cag gcc ttt gga cag 912
   Gly Met Thr Leu Arg Gly Ala Gly Gly Arg Leu Gln Ala Phe Gly Gln
   290 295 300
   cag aac aac aac cct aat ggc ttg act ccg aag cag gag atg ctg gcg 960
   Gln Asn Asn Asn Pro Asn Gly Leu Thr Pro Lys Gln Glu Met Leu Ala
   305 310 315 320
   agg tct gct att aag tat tgg gtg gag agg cac aaa cac gtc gtc aga 1008
   Arg Ser Ala Ile Lys Tyr Trp Val Glu Arg His Lys His Val Val Arg
   325 330 335
   ttg gta gca tcg ata gga gac acg tat ggt act gcc ctg ctg ttc cac 1056
   Leu Val Ala Ser Ile Gly Asp Thr Tyr Gly Thr Ala Leu Leu Phe His
   340 345 350
   atg ttg gtg tca act ata aca ctc act cta ttg gct tac cag gct act 1104
   Met Leu Val Ser Thr Ile Thr Leu Thr Leu Leu Ala Tyr Gln Ala Thr
   355 360 365
   aag atc aat gga att aat gtc tac gcg ttc agt act att ggc tac ctc 1152
   Lys Ile Asn Gly Ile Asn Val Tyr Ala Phe Ser Thr Ile Gly Tyr Leu
   370 375 380
   agc tat acc ctg gga cag gtg ttc cat ttc tgt atc ttc ggg aac agg 1200
   Ser Tyr Thr Leu Gly Gln Val Phe His Phe Cys Ile Phe Gly Asn Arg
   385 390 395 400
   ctt att gaa gag agt tcc tca gtg atg gaa gcc gcc tac tcc tgt cag 1248
   Leu Ile Glu Glu Ser Ser Ser Val Met Glu Ala Ala Tyr Ser Cys Gln
   405 410 415
   tgg tac gac ggc tcc gag gaa gca aag acc ttc gtc cag atc gtg tgc        1296
   Trp Tyr Asp Gly Ser Glu Glu Ala Lys Thr Phe Val Gln Ile Val Cys
               420                 425                 430
   cag caa tgc cag aaa gcc atg agc atc tcc ggg gcc aag ttc ttc acg        1344
   Gln Gln Cys Gln Lys Ala Met Ser Ile Ser Gly Ala Lys Phe Phe Thr
           435                 440                 445
   gtg tca ctt gat ctt ttt gct tcg gta ctt ggt gca gta gtt act tac        1392
   Val Ser Leu Asp Leu Phe Ala Ser Val Leu Gly Ala Val Val Thr Tyr
       450                 455                 460
   ttc atg gtg ttg gta caa ctc aag taa                                    1419
   Phe Met Val Leu Val Gln Leu Lys
   465                 470
<210> 18
   <211> 472
   <212> PRT
   <213> Spodoptera littoralis
<400> 18
   Met Thr Lys Val Lys Ala Gln Gly Leu Val Ser Asp Leu Met Pro Asn
   1               5                   10                  15
   Ile Lys Leu Met Gln Ala Ala Gly His Phe Leu Phe Asn Tyr His Ala
               20                  25                  30
   Glu Asn Gly Gly Met Ser Gly Leu Leu Arg Lys Ile Tyr Ala Ser Thr
           35                  40                  45
   His Ala Ile Leu Ile Thr Ile His Phe Ala Cys Met Gly Ile Asn Met
       50                  55                  60
   Ala Gln Tyr Ser Asp Glu Val Asn Glu Leu Thr Ala Asn Thr Ile Thr
   65                  70                  75                  80
   Val Leu Phe Phe Thr His Thr Ile Ile Lys Leu Gly Phe Phe Ala Leu
                   85                  90                  95
   Asn Ser Lys Ser Phe Tyr Arg Thr Leu Ala Val Trp Asn Gln Ser Asn
               100                 105                 110
   Ser His Pro Leu Phe Thr Glu Ser Asp Ala Arg Tyr His Gln Ile Ala
           115                 120                 125
   Leu Thr Lys Met Arg Arg Leu Leu Tyr Phe Ile Cys Gly Met Thr Val
       130                 135                 140
   Leu Ser Val Val Ser Trp Val Thr Leu Thr Phe Phe Gly Glu Ser Val
   145                 150                 155                 160
   Arg Leu Ile Thr Ser Lys Glu Thr Asn Glu Thr Leu Thr Glu Val Ala
                   165                 170                 175
   Pro Arg Leu Pro Leu Lys Ala Trp Tyr Pro Phe Asn Ala Met Ser Gly
               180                 185                 190
   Thr Thr Tyr Ile Ile Ala Phe Ala Phe Gln Val Tyr Trp Leu Leu Phe
           195                 200                 205
   Ser Met Ala Ile Ala Asn Leu Met Asp Val Met Phe Cys Ser Trp Leu
       210                 215                 220
   Ile Phe Ala Cys Glu Gln Leu Gln His Leu Lys Ala Ile Met Lys Pro
   225                 230                 235                 240
   Leu Met Glu Leu Ser Ala Ser Leu Asp Thr Tyr Arg Pro Asn Thr Ala
                   245                 250                 255
   Glu Leu Phe Arg Ala Ser Ser Thr Glu Lys Ser Glu Lys Ile Pro Asp
               260                 265                 270
   Thr Val Asp Met Asp Ile Arg Gly Ile Tyr Ser Thr Gln Gln Asp Phe
           275                 280                 285
   Gly Met Thr Leu Arg Gly Ala Gly Gly Arg Leu Gln Ala Phe Gly Gln
       290                 295                 300
   Gln Asn Asn Asn Pro Asn Gly Leu Thr Pro Lys Gln Glu Met Leu Ala
   305                 310                 315                 320
   Arg Ser Ala Ile Lys Tyr Trp Val Glu Arg His Lys His Val Val Arg
                   325                 330                 335
   Leu Val Ala Ser Ile Gly Asp Thr Tyr Gly Thr Ala Leu Leu Phe His
               340                 345                 350
   Met Leu Val Ser Thr Ile Thr Leu Thr Leu Leu Ala Tyr Gln Ala Thr
           355                 360                 365
   Lys Ile Asn Gly Ile Asn Val Tyr Ala Phe Ser Thr Ile Gly Tyr Leu
       370                 375                 380
   Ser Tyr Thr Leu Gly Gln Val Phe His Phe Cys Ile Phe Gly Asn Arg
   385                 390                 395                 400
   Leu Ile Glu Glu Ser Ser Ser Val Met Glu Ala Ala Tyr Ser Cys Gln
                   405                 410                 415
   Trp Tyr Asp Gly Ser Glu Glu Ala Lys Thr Phe Val Gln Ile Val Cys
               420                 425                 430
   Gln Gln Cys Gln Lys Ala Met Ser Ile Ser Gly Ala Lys Phe Phe Thr
           435                 440                 445
   Val Ser Leu Asp Leu Phe Ala Ser Val Leu Gly Ala Val Val Thr Tyr
   450 455 460
   Phe Met Val Leu Val Gln Leu Lys
   465 470
<210> 19
   <211> 1053
   <212> DNA
   <213> spodoptera littoralis
<220>
   <221> CDS
   <222> (1)..(1053)
   <223> PBAN receptor encoding sequence
<400> 19
   atg aca ttg tca gcg ccc ccg atc gac gag ttt gag gac ccg ttc gtc      48
   Met Thr Leu Ser Ala Pro Pro Ile Asp Glu Phe Glu Asp Pro Phe Val
   1               5                   10                  15
   gtg atg aac acg acg aat gtg tcg agt cac ccg gcc gct tat gac gag      96
   Val Met Asn Thr Thr Asn Val Ser Ser His Pro Ala Ala Tyr Asp Glu
               20                  25                  30
   cca tac acg ctc gat ctg gtg gtg cca ctc acc gtc acc tac gtg gtg     144
   Pro Tyr Thr Leu Asp Leu Val Val Pro Leu Thr Val Thr Tyr Val Val
           35                  40                  45
   ata ttc gta gcc ggc atc ctg ggg aac acc agc acc tgt gtg gtc att     192
   Ile Phe Val Ala Gly Ile Leu Gly Asn Thr Ser Thr Cys Val Val Ile
       50                  55                  60
   gcg agg aac cgc tct atg cac acc gct aca aac ttt tac ctc ttc agt     240
   Ala Arg Asn Arg Ser Met His Thr Ala Thr Asn Phe Tyr Leu Phe Ser
   65                  70                  75                  80
   tta gca att tcg gat ctc ata ttg tta gta tgt ggt ctg ccg ttt gag     288
   Leu Ala Ile Ser Asp Leu Ile Leu Leu Val Cys Gly Leu Pro Phe Glu
                   85                  90                  95
   gtg cac aga tta tgg aac ccg gac acg tac ccg ttg ggc gag gct cac     336
   Val His Arg Leu Trp Asn Pro Asp Thr Tyr Pro Leu Gly Glu Ala His
               100                 105                 110
   tgc atc gcc att ggg tta gcg tcg gag act tca gct aac gcg aca gtg     384
   Cys Ile Ala Ile Gly Leu Ala Ser Glu Thr Ser Ala Asn Ala Thr Val
           115                 120                 125
   ttg aca ata aca gcg ttt acg gtg gag cgg tac att gcg ata tgt aga     432
   Leu Thr Ile Thr Ala Phe Thr Val Glu Arg Tyr Ile Ala Ile Cys Arg
       130                 135                 140
   cct ttc atg tcg cac acc atg tcg aag ctg tca cgg gct gtg cgg ttt     480
   Pro Phe Met Ser His Thr Met Ser Lys Leu Ser Arg Ala Val Arg Phe
   145                 150                 155                 160
   ata ata gct att tgg gtg ttt gcg ttg tgt act gcg gta cca caa gcg     528
   Ile Ile Ala Ile Trp Val Phe Ala Leu Cys Thr Ala Val Pro Gln Ala
                   165                 170                 175
   atg cag ttc ggt ata gtg tcg tat gtg gat aat ggt cag aat gtg agc     576
   Met Gln Phe Gly Ile Val Ser Tyr Val Asp Asn Gly Gln Asn Val Ser
               180                 185                 190
   gcg tgc aca gtg aag ggt gtg ggt gtt cat caa gtg ttc gtt ata tct        624
   Ala Cys Thr Val Lys Gly Val Gly Val His Gln Val Phe Val Ile Ser
           195                 200                 205
   agt ttc gtg ttc ttt gtg gtg cct atg tcg atg ata tca gtg ttg tac        672
   Ser Phe Val Phe Phe Val Val Pro Met Ser Met Ile Ser Val Leu Tyr
       210                 215                 220
   gcg ttg atc ggc att aag ttg cgg acc tca cga gtg ttg cat cca gtg        720
   Ala Leu Ile Gly Ile Lys Leu Arg Thr Ser Arg Val Leu His Pro Val
   225                 230                 235                 240
   aag aag ctc tcg gtg gac agt aac gaa aga gcg agt gga cag atg cag        768
   Lys Lys Leu Ser Val Asp Ser Asn Glu Arg Ala Ser Gly Gln Met Gln
                   245                 250                 255
   tac agg aat gga gca tca cag agg aga gtc att aga atg ctg gtt gcg        816
   Tyr Arg Asn Gly Ala Ser Gln Arg Arg Val Ile Arg Met Leu Val Ala
               260                 265                 270
   gtg gcg ctg tca ttc ttc att tgc tgg gcg ccc ttc cac gtg caa agg        864
   Val Ala Leu Ser Phe Phe Ile Cys Trp Ala Pro Phe His Val Gln Arg
           275                 280                 285
   ttg ctg gcc atc tac ggg aaa agt tta gaa cac cct tct gat act ttc        912
   Leu Leu Ala Ile Tyr Gly Lys Ser Leu Glu His Pro Ser Asp Thr Phe
       290                 295                 300
   tac ttg gtc tac atc gtg ctg acg ttt ttg tcg gga gtg cta tat ttc        960
   Tyr Leu Val Tyr Ile Val Leu Thr Phe Leu Ser Gly Val Leu Tyr Phe
   305                 310                 315                 320
   ctg tca acc gcc atc aac ccg ttc ctg tac aac atc atg tcg aat aag       1008
   Leu Ser Thr Ala Ile Asn Pro Phe Leu Tyr Asn Ile Met Ser Asn Lys
                   325                 330                 335
   ttt aga aac gcc ttc aag gtg agt ttt tgt tac att cat gat tga           1053
   Phe Arg Asn Ala Phe Lys Val Ser Phe Cys Tyr Ile His Asp
               340                 345                 350
<210> 20
   <211> 350
   <212> PRT
   <213> Spodoptera littoralis
<400> 20
   Met Thr Leu Ser Ala Pro Pro Ile Asp Glu Phe Glu Asp Pro Phe Val
   1               5                   10                  15
   Val Met Asn Thr Thr Asn Val Ser Ser His Pro Ala Ala Tyr Asp Glu
               20                  25                  30
   Pro Tyr Thr Leu Asp Leu Val Val Pro Leu Thr Val Thr Tyr Val Val
           35                  40                  45
   Ile Phe Val Ala Gly Ile Leu Gly Asn Thr Ser Thr Cys Val Val Ile
       50                  55                  60
   Ala Arg Asn Arg Ser Met His Thr Ala Thr Asn Phe Tyr Leu Phe Ser
   65                  70                  75                  80
   Leu Ala Ile Ser Asp Leu Ile Leu Leu Val Cys Gly Leu Pro Phe Glu
                   85                  90                  95
   Val His Arg Leu Trp Asn Pro Asp Thr Tyr Pro Leu Gly Glu Ala His
               100                 105                 110
   Cys Ile Ala Ile Gly Leu Ala Ser Glu Thr Ser Ala Asn Ala Thr Val
           115                 120                 125
   Leu Thr Ile Thr Ala Phe Thr Val Glu Arg Tyr Ile Ala Ile Cys Arg
       130                 135                 140
   Pro Phe Met Ser His Thr Met Ser Lys Leu Ser Arg Ala Val Arg Phe
   145                 150                 155                 160
   Ile Ile Ala Ile Trp Val Phe Ala Leu Cys Thr Ala Val Pro Gln Ala
                   165                 170                 175
   Met Gln Phe Gly Ile Val Ser Tyr Val Asp Asn Gly Gln Asn Val Ser
               180                 185                 190
   Ala Cys Thr Val Lys Gly Val Gly Val His Gln Val Phe Val Ile Ser
           195                 200                 205
   Ser Phe Val Phe Phe Val Val Pro Met Ser Met Ile Ser Val Leu Tyr
       210                 215                 220
   Ala Leu Ile Gly Ile Lys Leu Arg Thr Ser Arg Val Leu His Pro Val
   225                 230                 235                 240
   Lys Lys Leu Ser Val Asp Ser Asn Glu Arg Ala Ser Gly Gln Met Gln
                   245                 250                 255
   Tyr Arg Asn Gly Ala Ser Gln Arg Arg Val Ile Arg Met Leu Val Ala
               260                 265                 270
   Val Ala Leu Ser Phe Phe Ile Cys Trp Ala Pro Phe His Val Gln Arg
           275                 280                 285
   Leu Leu Ala Ile Tyr Gly Lys Ser Leu Glu His Pro Ser Asp Thr Phe
       290                 295                 300
   Tyr Leu Val Tyr Ile Val Leu Thr Phe Leu Ser Gly Val Leu Tyr Phe
   305                 310                 315                 320
   Leu Ser Thr Ala Ile Asn Pro Phe Leu Tyr Asn Ile Met Ser Asn Lys
                   325                 330                 335
   Phe Arg Asn Ala Phe Lys Val Ser Phe Cys Tyr Ile His Asp
               340                 345                 350
<210> 21
   <211> 944
   <212> DNA
   <213> Musca domestica
<220>
   <221> misc_feature
   <222> (1)..(944)
   <223> Nucleotidic sequence of the Octopamine receptor of Musca domestica
<400> 21
   tgggcgataa ccgatccaat aaactatgct caaaagcgga cagttggcag agttttgctg      60
   ttaatagctg gtgtctggat tttatcgcta ctcatcagtt cgccaccgtt ggttggttgg     120
   aatgattggc cggaagagtt taccagtgcc actccttgcg aactaaattc tgatcctggc     180
   tacgtaattt attcttcact gggatcattc ttcattccat taatcataat gacattggtt     240
   tatatagaaa tatttgttgc tacccggaga cgactacgag aaagggcgca ggcggcaaag     300
   ataaacacaa ttgccacacg atctgtcagc gctgatgtag caagcactac tgtgacggca     360
   aatcaaacgg gaaatggcac catatccatt ttgtgttaca gcgtcatttg ttgctgccgc     420
   aactcgacgc cattctctac aacgccgatg atacaaaacg accaagagtc cgaaagtagc     480
   gaaacaaatg ccaacaacga aagtacaaga gttcaaaatc aaacagatac caagacaact     540
   gtggcttcac agcgaaaaag gacccgaaga gccaaaatca aagactccat taaacatgga     600
   aaactaaagg ctcggtcagg tcatacattt aacgaccacc cagaagtaca gaaatctaag     660
   cagataggaa gtggtgcgga aagtcttcta ctggcaccca atgccaaagg cacagaaaat     720
   gaaaatcaag aaatatctac tgagagcggc agcgacgcaa aaggttgcat gcaagtgtgt     780
   ggaagtcgga acgacgccaa tgaaatctca gccggtgacg gcgaggaaga aaatgtgtct     840
   ctgaaaatta ccccacccca gtcgtctgta ggcgcaacgt cgttgcaaac agttccattg     900
   caaaagaaac cggggggcgt atatcagttc atcgaagaaa aaca                       944
<210> 22
   <211> 314
   <212> PRT
   <213> Musca domestica
<220>
   <221> misc_feature
   <222> (1)..(314)
   <223> Protein sequence of the Octopamine receptor if Musca domestica
<400> 22
   Trp Ala Ile Thr Asp Pro Ile Asn Tyr Ala Gln Lys Arg Thr Val Gly
   1               5                   10                  15
   Arg Val Leu Leu Leu Ile Ala Gly Val Trp Ile Leu Ser Leu Leu Ile
               20                  25                  30
   Ser Ser Pro Pro Leu Val Gly Trp Asn Asp Trp Pro Glu Glu Phe Thr
   35 40 45
   Ser Ala Thr Pro Cys Glu Leu Asn Ser Asp Pro Gly Tyr Val Ile Tyr
   50 55 60
   Ser Ser Leu Gly Ser Phe Phe Ile Pro Leu Ile Ile Met Thr Leu Val
   65 70 75 80
   Tyr Ile Glu Ile Phe Val Ala Thr Arg Arg Arg Leu Arg Glu Arg Ala
   85 90 95
   Gln Ala Ala Lys Ile Asn Thr Ile Ala Thr Arg Ser Val Ser Ala Asp
   100 105 110
   Val Ala Ser Thr Thr Val Thr Ala Asn Gln Thr Gly Asn Gly Thr Ile
   115 120 125
   Ser Ile Leu Cys Tyr Ser Val Ile Cys Cys Cys Arg Asn Ser Thr Pro
   130 135 140
   Phe Ser Thr Thr Pro Met Ile Gln Asn Asp Gln Glu Ser Glu Ser Ser
   145 150 155 160
   Glu Thr Asn Ala Asn Asn Glu Ser Thr Arg Val Gln Asn Gln Thr Asp
   165 170 175
   Thr Lys Thr Thr Val Ala Ser Gln Arg Lys Arg Thr Arg Arg Ala Lys
   180 185 190
   Ile Lys Asp Ser Ile Lys His Gly Lys Leu Lys Ala Arg Ser Gly His
   195 200 205
   Thr Phe Asn Asp His Pro Glu Val Gln Lys Ser Lys Gln Ile Gly Ser
   210 215 220
   Gly Ala Glu Ser Leu Leu Leu Ala Pro Asn Ala Lys Gly Thr Glu Asn
   225 230 235 240
   Glu Asn Gln Glu Ile Ser Thr Glu Ser Gly Ser Asp Ala Lys Gly Cys
   245 250 255
   Met Gln Val Cys Gly Ser Arg Asn Asp Ala Asn Glu Ile Ser Ala Gly
   260 265 270
   Asp Gly Glu Glu Glu Asn Val Ser Leu Lys Ile Thr Pro Pro Gln Ser
   275 280 285
   Ser Val Gly Ala Thr Ser Leu Gln Thr Val Pro Leu Gln Lys Lys Pro
   290 295 300
   Gly Gly Val Tyr Gln Phe Ile Glu Glu Lys
   305 310
<210> 23
   <211> 1338
   <212> DNA
   <213> Leptinotarsa decemlineata
<220>
   <221> misc_feature
   <222> (1)..(1338)
   <223> Octopamine receptor Leptinotarsa decemlineata encoding sequence
<400> 23
   atgggagaag aacacaacaa ttgttccgat gagccgtacg gcaatttctc caccatattc      60
   ggttcaacag acattcctga atgggaagtg atcatcacaa taggaacttt gtcccttttc     120
   atcctactca cagtaatagg aaacgtcctc gtgattttga gcgtttttac ctacaagcca     180
   ttgcgaatag ttcagaattt tttcatcatc tctcttgctg tggccgacct gaccgtggct     240
   ctccttgtcc ttccatttaa tgtggcgtat accgttttag ggcgatggat atttggtatc     300
   catgtctgca aaatgtggct cacatcagac gtcttgtgct gcactgcctc cattttgaat     360
   ctttgtgcaa ttgctctcga caggttttgg gctataacag atcctataaa ctatgctcaa     420
   aaaaggacag ttaaacgagt actggttatg atagcaggcg tatgggtgct atcacttttg     480
   atcagttcac ctcctttgat tgggtggaat gactggccat catctgatgt attcgcagaa     540
   agacaaattt gtatgctcac aaaacgccaa ggttatgtga tttattcatc ttctggttcc     600
   ttttttatac ctctattcat catgacaata gtttatgtag aaatattcat tgcgacaaaa     660
   cgaaggctga gagaaaggtc ccaagcagcg aaaattaaca tatctaaaaa tatgagtaaa     720
   attaaaaaca aagacgatca tcgcgatggt gaaagcgtca gtagcgaaac caatcataac     780
   gaattcgaag cgaatggtaa atctagagat aaatctttga gaaaagacaa atcgagcgaa     840
   tctgataaaa atactcttca accgatattg actcatgaag attctctaac tgattttgga     900
   gacaactcat cctgctctca aaggaaagac tcgaatccct caaaatgtga gaataaggga     960
   atttgtagtt caaccactac cataacgatc tccaactcga aacctagtcc aaaagtaata    1020
   ctcagtgatc ccaataaaaa acctggattt gtatatcagt tcatagagga aaaacagaga    1080
   atatcacttt ctaaagagag acgagcagca agaacgcttg gtatcattat gggtgttttt    1140
   gtcgtatgct ggttaccctt tttccttatg tacgtgataa ttccgttctg tccgtcatgt    1200
   tgtccttcaa gagcattaac gaatgttatc acttggttgg gttatatcaa ctcaggactc    1260
   aatcccgtca tatataccat tttcaatatg gacttcagaa gggctttcag aaaactgtta    1320
   ggtctttctc aaacctaa                                                  1338
<210> 24
   <211> 445
   <212> PRT
   <213> Leptinotarsa decemlineata
<220>
   <221> misc_feature
   <222> (1)..(445)
   <223> Octopamine receptor Leptinotarsa decemlineata protein sequence
<400> 24
   Met Gly Glu Glu His Asn Asn Cys Ser Asp Glu Pro Tyr Gly Asn Phe
   1               5                   10                  15
   Ser Thr Ile Phe Gly Ser Thr Asp Ile Pro Glu Trp Glu Val Ile Ile
               20                  25                  30
   Thr Ile Gly Thr Leu Ser Leu Phe Ile Leu Leu Thr Val Ile Gly Asn
           35                  40                  45
   Val Leu Val Ile Leu Ser Val Phe Thr Tyr Lys Pro Leu Arg Ile Val
       50                  55                  60
   Gln Asn Phe Phe Ile Ile Ser Leu Ala Val Ala Asp Leu Thr Val Ala
   65                  70                  75                  80
   Leu Leu Val Leu Pro Phe Asn Val Ala Tyr Thr Val Leu Gly Arg Trp
                   85                  90                  95
   Ile Phe Gly Ile His Val Cys Lys Met Trp Leu Thr Ser Asp Val Leu
               100                 105                 110
   Cys Cys Thr Ala Ser Ile Leu Asn Leu Cys Ala Ile Ala Leu Asp Arg
           115                 120                 125
   Phe Trp Ala Ile Thr Asp Pro Ile Asn Tyr Ala Gln Lys Arg Thr Val
       130                 135                 140
   Lys Arg Val Leu Val Met Ile Ala Gly Val Trp Val Leu Ser Leu Leu
   145                 150                 155                 160
   Ile Ser Ser Pro Pro Leu Ile Gly Trp Asn Asp Trp Pro Ser Ser Asp
                   165                 170                 175
   Val Phe Ala Glu Arg Gln Ile Cys Met Leu Thr Lys Arg Gln Gly Tyr
               180                 185                 190
   Val Ile Tyr Ser Ser Ser Gly Ser Phe Phe Ile Pro Leu Phe Ile Met
           195                 200                 205
   Thr Ile Val Tyr Val Glu Ile Phe Ile Ala Thr Lys Arg Arg Leu Arg
       210                 215                 220
   Glu Arg Ser Gln Ala Ala Lys Ile Asn Ile Ser Lys Asn Met Ser Lys
   225                 230                 235                 240
   Ile Lys Asn Lys Asp Asp His Arg Asp Gly Glu Ser Val Ser Ser Glu
   245 250 255
   Thr Asn His Asn Glu Phe Glu Ala Asn Gly Lys Ser Arg Asp Lys Ser
   260 265 270
   Leu Arg Lys Asp Lys Ser Ser Glu Ser Asp Lys Asn Thr Leu Gln Pro
   275 280 285
   Ile Leu Thr His Glu Asp Ser Leu Thr Asp Phe Gly Asp Asn Ser Ser
   290 295 300
   Cys Ser Gln Arg Lys Asp Ser Asn Pro Ser Lys Cys Glu Asn Lys Gly
   305 310 315 320
   Ile Cys Ser Ser Thr Thr Thr Ile Thr Ile Ser Asn Ser Lys Pro Ser
   325 330 335
   Pro Lys Val Ile Leu Ser Asp Pro Asn Lys Lys Pro Gly Phe Val Tyr
   340 345 350
   Gln Phe Ile Glu Glu Lys Gln Arg Ile Ser Leu Ser Lys Glu Arg Arg
   355 360 365
   Ala Ala Arg Thr Leu Gly Ile Ile Met Gly Val Phe Val Val Cys Trp
   370 375 380
   Leu Pro Phe Phe Leu Met Tyr Val Ile Ile Pro Phe Cys Pro Ser Cys
   385 390 395 400
   Cys Pro Ser Arg Ala Leu Thr Asn Val Ile Thr Trp Leu Gly Tyr Ile
   405 410 415
   Asn Ser Gly Leu Asn Pro Val Ile Tyr Thr Ile Phe Asn Met Asp Phe
   420 425 430
   Arg Arg Ala Phe Arg Lys Leu Leu Gly Leu Ser Gln Thr
   435 440 445
<210> 25
   <211> 39
   <212> DNA
   <213> Artificial
<220>
   <223> MdOctR Forward Primer
<400> 25
   tgggcatcga tatcacatcg gatcccatcg atatcaatc 39
<210> 26
   <211> 38
   <212> DNA
   <213> Artificial
<220>
   <223> MdOctR Forward Primer
<400> 26
   caatcgacat cgacagaaat cgacatcgcc catatgat 38

## Description

The present invention relates to a new group of pesticides consisting of inactivated microorganisms containing double-strand RNA molecules (dsRNA) corresponding to receptor genes coupled with G proteins (GPCR) whose functioning is vital for phytophagous invertebrates (insects, mites and molluscs) or for infesting or in any case harmful organisms for the health of human beings, and to a method for the preparation of said microorganisms.

The discovery of RNA interference (RNAi) as a powerful and simple gene silencing method has aroused the attention of the whole scientific community. The ubiquity of the phenomenon has allowed it to be studied in many species, as the process has been preserved during its evolution.

This is a gene silencing mechanism whereby various double-strand RNA fragments (dsRNA) are capable of interfering and extinguishing the gene expression (Brantl S., 2002). Through an enzyme called *dicer,* the dsRNA sequence is cut into fragments having a shorter length (19-21 pairs of bases) (Hamilton and Baulcombe, 1999). The short dsRNA fragment (called *short interfering RNA,* or siRNA) is associated with an enzymatic complex called RISC (*RNA-interference silencing complex*). The dsRNA is opened, probably by a helicase: only the antisense RNA strand remains associated with RISC, whereas the sense strand is degraded. The RISC complex is capable of recognizing among the many mRNAs present in the cytosol, that which is complementary to the antisense RNA fragment associated with the same complex. If the pairing between siRNA and mRNA is perfect (or almost perfect), a component of RISC (called *argonaute protein*) is capable of effecting a cut on the mRNA (Hammond et al., 2001). The two resulting mRNA fragments, one hood-free at 5' and the other poly-A tail-free at 3', are thus rapidly degraded by the RNAsi of the same cell.

Another common protein, although not universally present in the RNAi machinery, is RNA-dependant RNA polymerase (RdRP) which synthesizes dsRNA starting from single-helix RNA pattern to create the amplification mechanism of RNAi.

Thanks to experiments on *C. elegans,* numerous genes involved in RNAi have been identified, of which the homologues in *Drosophila,* plants and fungi, were then identified. These studies have demonstrated that the phenomena which were first classified as PTGS (post-transcriptional gene silencing), quelling or co-suppression, are all part of a single process whose roots are embedded in a single ancestral mechanism. Silencing experiments were carried out using dsRNA on pathogens and phytophagous organisms of plants of agrarian interest to inhibit the expression of some of their vital genes. Micro-injections of dsRNA into the final stage larvae and adults of *Tribolium castaneum* beetles allowed genes to be silenced together with a study of their function (Tomoyasu and Denell, 2004). Various researchers performed assays with larvae of *Diabrotica virgifera virgifera* LeConte fed with a synthetic diet enriched with specific dsRNA to indentify essential genes for the vitality of the insect (Baum *et al*., 2007). These assays led to the identification of 14 essential genes whose inactivation due to interference, causes larval death. The same authors have demonstrated that transgenic corn plants expressing one of these dsRNAs show high protection levels, comparable to those obtained with transgenic plants expressing *Bt* toxin.

For the control of pathogen agents and phytophagous organisms, the selection of the gene target to be inactivated is of fundamental importance. For this purpose, the receptors coupled with G proteins (GPCR), a vast group of proteins structurally and functionally similar to each other, represent excellent targets as they exert functions of vital importance in all eukaryotic organisms (Hill, 2006). They are composed of a single polypeptide chain which crosses the membrane seven times having the amino-terminal end extracellular and the carboxy-terminal end intracellular; after binding with extracellular ligands (peptides, small molecules, ions, light and aromatic compounds), the receptor is activated by triggering a response in the cell which is translated into the production of a second messenger (cAMP, Ca²⁺, cGMP, IP₃).

Most insects of agrarian interest have numerous GPCRs, the majority of which are fundamental for the correct running of their vital functions. The most widely-studied species, whose genome has been completely sequenced and for which there is considerable information on the development biology, are the dipterans *Drosophila melanogaster, Anopheles gambiae* and the lepidopter *Bombyx mori. Drosophila melanogaster* is certainly the most widely-studied arthropod and is considered the model organism for the study of harmful insects. Approximately 270 GPCRs have been identified in the genome of *Drosophila,* grouped into 5 families.

As far as molluscs are concerned, there is no information on GPCRs of species harmful for agriculture, such as Helix *spp.,* Cepacea *spp.,* Arion *spp.* and Deroceras *spp.* Information is available in data banks on GPCR receptors of more widely-studied species. *Aplysia californica,* for example, represents a model species for the study of the nervous system and electrophysiology in molluscs, and parts of the genome of this species have therefore been sequenced. Other GPCR sequences have been identified in species of the kinds *Lymnea, Crassostrea, Haliotis, Octopus* e *Loligo.*

In conclusion, interference by RNAi on the GPCRs of species of insects or other harmful organisms appears to be extremely specific, as the gene homology percentage between the GPCRs of invertebrates and those of mammals never exceeds 20-30%. The percentage relating to the homology between the GPCRs of species belonging to the same order is also never complete (in insects for example, it is around 90%) and this guarantees discrimination between species of harmful organisms and those beneficial for the environment.

Most of the substances used as insecticides are currently molecules within a wide range, there is consequently an enormous demand for more selective methods which specifically interfere with the survival and fertility of harmful organisms alone, but which, at the same time, have a minimum or zero effect on other non-target organisms and on the environment.

The massive use of halogenated hydrocarbons, carbamates, pyrethroids and organo-phosphates against phytophagous insects, for example, has caused various toxic effects on the environment and on other non-target organisms, and has also generated resistance forms which have subsequently thwarted use against various species. Insecticides such as pyrethrins and pyrethroids (cypermethrin, tetra- and deltamethrin, bifenthrin), acetamidines (acetamiprid), organo-phosphates and nicotinoids, are also used against insects infesting household environments (flies, mosquitoes, ants and cockroaches). Although most of these products are used limitedly, they can cause toxicity, in both human beings and pets, and also in the environment. Pyrethroids, for example, can cause convulsions and vertigo, as far as serious nervous disturbances and a reduction in fertility. Cases are known of human contacts with organo-phosphate products which have caused stinging of the mucous membranes, breathing difficulties and abdominal cramp. Furthermore, nicotinoids are extremely toxic for aquatic organisms and are causing significant reductions in the populations of bee farms.

Zheng et al. (Gene, vol. 393, no. 1-2, (2007), p. 20-30) discloses the expression and characterization of PBAN-R peptides cloned from *S. Littoralis* larvae.

Tian Honggang et al. (Plos One, vol. 4, no. 7, E6225, (2009), p. 1-13) discloses that the ingestion of bacterially expressed dsRNA can specifically inhibit transcription of a targeted gene in the lepidopteran insect *S. exigua.*

WO 03/052078 discloses isolated nucleic acid encoding insect G protein-coupled receptor (GPCR) polypeptides, isolated insect GPCR polypeptides, and uses thereof.

Baum James A. et al. (Nature Biotechnology, (2007) Vol. 25, n. 11, p. 1322-1326) discloses an insect control strategy of coleopteran insect pests through RNA interference process.

Considering the specificity of RNAi-mediated insecticidal effects, Price D R G et al. (Trends in Biotechnology, Elsevier Publications, vol.26, no.7, (2008), p. 393-400) discloses the RNAi-mediated crop protection against insects.

On the basis of what is specified above, the necessity is evident for availing of new methods and relative biological targets which allow specific interference with the survival and fertility of organisms harmful for plants and which infest household environments, which are more selective and which consequently have a lower environmental impact.

The authors of the present invention have prepared new pesticides and relative treatment and protection methods of plants, food products, human households, based on the use of deactivated microorganisms (bacteria, yeasts, fungi, protozoans or cells of higher organisms) containing in their interior molecules of dsRNA capable of inhibiting the functionality of a GPCR receptor which, once ingested by the phytophagous or infesting organism, causes its death or a reduction in its vitality. GPCR receptors can in fact be convenient targets for controlling harmful and infesting organisms as they have a gene sequence which is very specific for harmful organisms. The dsRNA molecules are consequently capable of inhibiting their functionality and cannot cause damage either to the plant treated or to non-target consumers of the same plant (i.e. human beings, other animals). These dsRNA molecules capable of inhibiting the functionality of a GPCR receptor are produced by specific microorganisms which, once deactivated, represent excellent vehicles for transporting the dsRNA as far as the digestive system of the harmful organism. The involucre, consisting of the dead microorganism (therefore no longer able to grow or reproduce itself) protects the dsRNA molecules from degradation, until they are ingested by the phytophagous insect, which feeds on plant tissues, or by an infesting organism which feeds on food products or scraps. The dsRNA molecules only exert their insecticide action when they penetrate inside the body of the specific insect against which they have been produced. The mortal effect or reduction in vitality is not exerted against other non-target organisms, even if they feed on the plant or accidentally ingest the microorganisms containing dsRNA. Each product contains specific dsRNA molecules for the target organism against which they are to act. One product however can also be effective against various species contemporaneously if it derives from the mixture of various preparations of microorganisms/dsRNAs, each one selective for a single species. This allows a significant control of the action and efficacy of the product, as it is strictly oriented towards the species to be struck, without interfering with any other non-target organism.

Once the product (i.e. deactivated microorganism containing dsRNA against GPCRs of interest) has been prepared, it can be dissolved in water (or in another water-based buffer solution) and sprayed onto the leaves and stem of vegetables (action against insects and other harmful phytophages), or mixed with appropriate baits (for insects infesting households, food products) or mixed with the excrements of farm animals (against insects infesting the environments of domestic animals), or sprayed on furniture or wooden structures (for xylophage insects), or administered in the environment, on the soil or in water ponds (against mosquito larvas).

In addition to being applied to agriculture, the invention is also applied for fighting all species of insects and mites which infest and damage human houses or domestic animals. Many species of insects in fact damage food products or crops stored before being transported and processed, infest human households creating the risk of transmitting diseases (flies, cockroaches, ants), or are a direct threat to architectural structures and objects of household use (termites, woodworm, moths). Furthermore, the technology described herein can also be used against all bothersome insects which, by molesting domestic animals, cause a significant reduction in the yield of zootechnic productions (flies, horseflies, mosquitoes).

As this technology is based on a product which must be ingested by the target organism, it can only be applied for fighting animals which chew the tissues of plants or which feed on scraps or food products. In the case of phytophages, the product can be easily sprayed on the leaves and stem of plants, whereas in the case of animals infesting household environments, the product can be mixed or sprayed on appropriate baits. The technology in question however cannot be applied for fighting plant-sucking insects which, by perforating the vegetable tissue to have access to its lymph, do not come into contact with the product sprayed on the surface.

The advantages which can be associated with the technology, object of the present invention, can be summarized as follows:
a) the selectivity, which can harm a certain animal pest, but not other organisms which make use of the same food (human beings or animals) or which accidentally come into contact with the product (benevolent animals which live in the same environment);
b) food safety, for the same reason as above, but also due to the fact that an RNA molecule can be easily degraded and does not remain for a long period in the environment;
c) potential absence of allergenic risk, as proteins or chemical substances which can be toxic or with antigenic properties are not used;
d) facility of use and application of the product which can be easily sprayed onto plants as if it were a simple chemical product.

An object of the present invention therefore relates to transgenic microorganisms transformed with at least one expression vector comprising a cDNA sequence encoding a GPCR receptor which is vital for a phytophagous animal or pest, or a portion thereof, said receptor being selected from the group consisting of allatostatin, diuretic hormone, octopamine/tyramine, PBAN peptide, adipokinetic hormone, neuropeptide, dopamine, serotonin, gonadotropin receptors, as well as olfactory or rhodopsin-like receptors, said microorganisms being characterized by the fact that they have been inactivated.

The term microorganism in the present invention comprises prokaryotic and eukaryotic cells selected from the groups which consists of bacteria, yeasts, fungi, protozoa, insect cells, mammalian cells or plant cells.
According to a particularly preferred embodiment of the invention, said microorganism is *E.coli.* According to alternative embodiments, said microorganism is a yeast, preferably selected from *Saccharomyces* and *Pichia.*

In a preferred embodiment, said microorganisms are transformed with a cDNA encoding a GPCR receptor which is vital for *Spodoptera littoralis* having a nucleotide sequence selected from the group consisting in allatostatin receptor C AlstCR (SEQ ID NO:1 encoding SEQ ID NO:2) allostatin A receptor, AlstAR (sequence FJ374689 encoding the sequence ACJ06649; SEQ ID NO: 9-SEQ ID NO: 10), diuretic hormone receptor, DHR (sequence FJ374690 encoding the sequence ACJ06650; SEQ ID NO:11-SEQ ID NO:12), adipokinetic hormone receptor, AKHR (SEQ ID NO:3 encoding SEQ ID NO:4), hormone receptor LGR1 (sequence FJ374692 encoding the sequence ACJ06652; SEQ ID NO:13-SEQ ID NO:14), receptor of octopamine/tyramine, Oct/TyrR (sequence FJ374691 encoding the sequence ACJ06651; SEQ ID NO:15-SEQ ID NO:16), olfactory receptor OR83b (sequence FJ374688 encoding the sequence ACJ06648; SEQ ID NO:17-SEQ ID NO:18) and the PBAN, PBAN-R peptide receptor (sequence ABD52277 encoding the sequence DQ407742; SEQ ID NO:19-SEQ ID NO:20).

In a further preferred embodiment, said microorganisms are transformed with a cDNA encoding a GPCR receptor vital for *Musca domestica* having the nucleotide sequence (SEQ ID NO:21) encoding the receptor of octopamine having SEQ ID NO:22.

Again according to another embodiment of the present invention, said microorganisms are transformed with a cDNA encoding a GPCR receptor vital for *Leptinotarsa decemlineata* having the nucleotide sequence (SEQ ID NO:23) encoding the receptor of octopamine having SEQ ID NO:24.

A further object of the present invention relates to a method for obtaining transgenic microorganisms containing at least one dsRNA capable of inhibiting the functionality of a GPCR receptor suitable for eliminating the attack of one or more phytophagous organisms or pests, comprising the following phases:
a) isolation of the cDNA nucleotide sequence encoding a receptor coupled with G proteins (GPCR), or portion thereof, which exerts functions of vital importance for the phytophagous organism or pest, selected from the group consisting in receptors for allatostatin, diuretic hormone, octopamine/tyramine, PBAN peptide, adipokinetic hormone, neuropeptide, dopamine, serotonin, gonadotropin, as well as olfactory or rhodopsin-like receptors;
b) construction of an appropriate expression vector comprising the cDNA nucleotide sequence encoding a GPCR receptor or portion thereof, as determined in step a); each expression vector is selected on the basis of the microorganism which is to be used as dsRNA vector, an operation which falls within the knowledge of an expert in the field;
c) transfer of said vector or recombinant plasmid into the cell of a microorganism selected from bacteria, yeasts, fungi, protozoa, or into a eukaryotic cell in culture selected from insect cells, mammalian cells or plant cells for dsRNA expression;
d) growth of the microorganisms or eukaryotic cell culture expressing dsRNA, until dense cultures are obtained;
e) spinning the cell culture to eliminate the culture medium, washing the pellet obtained and lyophilization to eliminate all of the liquid component;
f) inactivation of the microorganisms by heat or by other physical procedures.

According to the present invention, "dense culture" refers in the case of bacteria to cultures with an optical density higher than 0.6 DO, whereas in the case of eukaryotic cells a number of cells higher than 10⁷ per millilitre.

The present invention also relates to transgenic microorganisms containing at least one dsRNA capable of inhibiting the functionality of a GPCR receptor of a phytophage or pest which can be obtained with the preparation method indicated above.

A further object of the present invention relates to the use of the microorganisms as defined above, alone or associated with other strains of microorganisms transformed with a cDNA encoding a GPCR receptor vital for a different phytophage or pest, as pesticides against larvas and/or adult insects.

According to a preferred embodiment, the invention relates to the specific use of microorganisms (i.e. *E.coli*) transformed with a cDNA encoding a GPCR receptor vital for *Spodoptera littoralis* having a nucleotide sequence selected from the group consisting in AlstAR (sequence FJ374689 encoding the sequence ACJ06649; SEQ ID NO: 9- SEQ ID NO:10), AlstCR (SEQ ID NO:1-SEQ ID NO:2), DHR (sequence FJ374690 encoding the sequence ACJ06650; SEQ ID NO: 11-SEQ ID NO: 12), AKHR (SEQ ID NO:3-SEQ ID NO:4), LGR1 (sequence FJ374692 encoding the sequence ACJ06652; SEQ ID NO: 13-SEQ ID NO:14), Oct/TyrR (sequence FJ374691 encoding the sequence ACJ06651; SEQ ID NO:15-SEQ ID NO:16), OR83b (sequence FJ374688 encoding the sequence ACJ06648; SEQ ID NO:17- SEQ ID NO:18) and PBANR (sequence ABD52277 encoding the sequence DQ407742; SEQ ID NO:19-SEQ ID NO:20), as a pesticide for the eradication of the lepidopter *Spodoptera littoralis.*
Alternatively, the invention relates to the specific use of microorganisms (i.e. *E.coli*) transformed with a cDNA encoding a GPCR receptor vital for *Musca domestica* having the nucleotide sequence (SEQ ID NO:21) encoding the receptor of octopamine having SEQ ID NO:22, as pesticide for the eradication of the dipteran *Musca domestica.* According to a further preferred embodiment, the invention relates to the specific use of microorganisms (i.e. *E.coli*) transformed with a cDNA encoding a GPCR receptor vital for *Leptinotarsa decemlineata* having the nucleotide sequence (SEQ ID NO:23) encoding the receptor of octopamine having SEQ ID NO:24, as pesticide for the eradication of the coleopteran *Leptinotarsa decemlineata.*

The invention also relates to a pesticide composition comprising the transgenic microorganisms as defined above as active ingredient at a concentration ranging from 1,000 to 30,000 ppm, in the presence or absence of excipients and/or adjuvants in use in the agricultural field or pesticide formulations.

In an alternative embodiment, said transgenic microorganisms can be different strains of the same or of a different microorganism transformed with cDNA encoding a GPCR receptor vital for different phytophages and/or pests. The method according to the present invention, in fact, envisages the selection of one or more GPCR receptors as RNA interference target, said receptor must be vital for the phytophagous organism or pest against which it should act. In relation to the organism, different types of GPCR receptors can be used as target. Consequently, according to a particularly preferred embodiment of the invention, phases a)-g) of the method according to the invention can be effected using strains of microorganisms each expressing a dsRNA encoding a GPCR receptor or parts of it, vital for the various species of harmful organisms to be struck.

Preferably, the pesticide composition based on microorganisms expressing dsRNAs specific for a GPCR receptor vital for phytophages or pests, is formulated as a spray, powder, suspension of the microorganisms in aqueous solution, lyophilized preparation, paste. In the application against *Spodoptera littoralis,* for example, the product according to the invention can be formulated in the form of a powder which is dissolved in water and administered to the larvae (i.e. through the leaves of plants on which they normally feed). In applications against *Musca domestica,* on the other hand, the product can be mixed directly with food-bait or with excrements of farm animals, on which the larvas of the insect feed.

A further object of the present invention relates to a method for controlling and/or eliminating one or more phytophagous organisms in the adult or larval state for the treatment of plants of agronomical (such as for example cereals, solanaceous plants, vines, vegetables), ornamental (such as roses, etc.) environmental (such as conifers, linden trees, poplar trees, etc.) interest, which comprises spraying a pesticide composition as defined above on the leaves or stem of said plants or in the ground. The plants treated with the products deriving from the present invention for inhibiting the functionality of the GPCR receptors of insects can be used for the production of food destined for human beings or for zootechnic, but also for the production of biomass for industrial purposes (paper, fibres, pharmacological substances, biomasses for energy production or basic chemical substances, etc.).

Said phytophagous organisms are preferably selected from the group consisting of lepidopters (i.e. Spodoptera littoralis), dipterans (i.e. Musca domestica), coleopterans (i.e. Leptinotarsa decemlineata), isopterans, thysanopterans, orthopterans, hymenopterans, xylophagous insects, among molluscs gasteropodes and mites. According to preferred embodiments, the herbivorous organism or pest in the adult or larval state can be selected from the following species (grouped according to the damage they cause):

### 1) Insects harmful for fruit plants:

a) LEPIDOPTERS: Prays citri (flower moth), Phyllocnistis citrulla (citrus-leaf miner), Leucoptera malifoliella (pear-leaf blister moth), Phyllonorycter blancardella (pnigalio Schrank), Cydia pomonella (apple worm), Cydia molesta (oriental peach moth), Cossus cossus (red woodworm);
b) COLEOPTERANS: Otiorhynchus cribricollis (otiorhynchus), Anomala spp. (anomala), Melolontha Melolontha (May-bug), Scolytus spp. (scolytus of almond and peach trees), Curculio spp. (curculionides);
c) DIPTERA: Ceratitis capitata (Mediterranean fruit fly), Rhagoletis cerasi (cherry fly);

### 2) Insects harmful for olive trees:

a) LEPIDOPTERS: Prays oleae (olive moth), Zeuzera pyrina (yellow goat moth);
b) COLEOPTERANS: Otiorrhynchus cribricollis (otiorhynchus);
c) DIPTERA: Bactrocera (=Dacus) oleae (olive moth);
d) ISOPTERANS: Kalotermes flavicollis (yellow neck kalotermes);

### 3) Insects harmful for vines:

a) LEPIDOPTERS: Lobesia botrana (vine moth);
b) COLEOPTERANS: Anomala vitis (Anomala);
c) DIPTERA: Ceratitis capitata (Mediterranean fruit fly) THYSANOPTERA: Frankliniella occidentalis (american thunderbug);

### 4) Insects harmful for cereals:

a) ORTHOPTERANS: Dociostaurus maroccanus (cross locust);
b) THYSANOPTERANS: Limothrips cerealium (wheat thrips),
c) LEPIDOPTERS: Ostrinia nubilalis (European corn borer), Sesamia spp (corn and sorghum sesamia), Helicoverpa zea (cotton worm);
d) COLEOPTERANS: Agriotes lineatus (elater);
e) DIPTERA: Mayetiola spp (wheat and oat gall-midge), Tipula oleracea (crane-fly);

### 5) Insects harmful for herbaceous plants and greens:

a) ORTHOPTERANS: Dociostaurus maroccanus (Cruciate locust), Locusta migratoria (locust), Gryllotalpa gryllotalpa (mole cricket), Gryllus spp (spotted country cricket);
b) THYSANOPTERANS: Frankliniella occidentalis (American thunderfly) ;
c) LEPIDOPTERS: Autographa gamma (plusia gamma), Mamestra brassicae (brassica noctuid), Spodoptera littoralis (spodoptera), Heliotis virescens (tabacco noctuid), Helicoverpa armigera (tomato noctuid), Pieris spp (cabbage butterfly and cabbage white), Tuta absoluta (tomato moth), Helicoverpa zea (cotton worm);
d) COLEOPTERANS: Leptinotarsa decemlineata (Potato-beetle), Agriotes lineatus (click-beetle), Othiorrynchus spp (Othiorrynchus);

### 6) Insects and mites infesting domestic environments:

1) ORTHOPTERANS: Blatta orientalis (black-beetle or cockroach), American Periplaneta (red cockroach or American cockroach), German Blattella (grey cockroach), Supella longipalpa, (wood black-beetle);
2) DIPTERAN: Musca domestica (common fly), Fannia canicularis (small house fly), Stomoxys calcitrans (haematophagous horsefly), Tabanus bovinus (gadfly), Sarcofaga carnaria (Sarcophagidae fly), Calliphora spp (blue fly) ande Lucilia spp (green fly); Culex pipiens (common mosquito), Aedes aegypti (tiger Egyptian mosquito), Aedes albopictus (tiger mosquito), Anopheles gambie (malaria anopheles);
3) HYMENOPTERA: Monomorium pharaonis (pharaonis ant), Iridomyrmex humilis (Argentinean ant), Lasius niger (black ant), Pheidole pallidula (red ant), Crematogaster scutellaris (bulldog ant);
4) LEPIDOPTERS and COLEOPTERANS, which feed on food products: Plodia interpunctella (food moth), Lasioderma serricorne (tobacco woodworm), Stegobium paniceum (bread woodworm), Sitophilus oryzae (rise-weevil), Tribolium spp (caltrops); or which feed on fabrics (wool, silk, cotton): Tineola bisselliella (tineid), Anthrenus spp and Attagenus spp (carpet coleopterans).
5) XYLOPHAGOUS INSECTS, which perforate and feed on furniture wood, works of art, beams and various structures: Hylotrupes bajulus (house-serow), Anobium punctatum (woodworm), Kalotermes flavicollis and, above all, Reticulitermes lucifugus (white ants);
6) MITES RESPONSIBLE FOR ALLERGIES: Dermatophagoides spp and Glycyphagus domesticus (powder mite).
7) PHYTOPHAGOUS MOLLUSCS: Helix spp., Cepaea spp (snails); Arion spp and Deroceras spp (slugs).

According to a preferred embodiment in which said phytophagous organism is *Spodoptera littoralis,* the nucleotide sequence of the cDNA encoding a GPCR receptor or a portion thereof, is selected from the group which consists in AlstAR (sequence FJ374689 encoding the sequence ACJ06649; SEQ ID NO: 9), AlstCR (SEQ ID NO:1), DHR (sequence FJ374690 encoding the sequence ACJ06650; SEQ ID NO: 11), AKHR (SEQ ID NO:3), LGR1 (sequence FJ374692 encoding the sequence ACJ06652; SEQ ID NO: 13), Oct/TyrR (sequence FJ374691 encoding the sequence ACJ06651; SEQ ID NO: 15), OR83b (sequence FJ374688 encoding the sequence ACJ06648; SEQ ID NO:17) and PBANR (sequence ABD52277 encoding the sequence DQ407742; SEQ ID NO:19).

With respect to other species of lepidopters, such as for example *Heliotis virescens,* the authors have found that the GPCR receptors homologous to those of allatostatin C and the diuretic hormone of *Spodoptera* could represent the election targets for this type of herbivorous insect.

In a further preferred embodiment in which said phytophagous organism is *Musca domestica,* the cDNA nucleotide sequence encoding a GPCR receptor or a portion thereof has the nucleotide sequence (SEQ ID NO:21) encoding the receptor of octopamine having SEQ ID NO:22. According to a further embodiment of the present invention in which said phytophagous organism is *Leptinotarsa decemlineata,* the cDNA nucleotide sequence encoding a GPCR receptor or a portion thereof has the nucleotide sequence (SEQ ID NO:23) encoding the receptor of octopamine having SEQ ID NO:24.

Furthermore, the technology in question can also be used for protecting products deriving from the agrarian or food industry. The stored crops, awaiting processing or sale, can undergo the attack of saprophyte organisms. Food products stored in non-cooled places can also be protected from insects and other arthropods with the use of the technology in question. Finally, the technology can be applied in house-holding and veterinary environments against all harmful organisms which infest and can be a threat to the health of human beings, products of human beings and domestic animals.

Finally, the invention relates to a method for the control and/or elimination of pests in the adult or larval state from an environment comprising the application of a pesticide composition in powder form, in solution or by spraying on a surface selected from wood, fabrics or soil, water well, excrements of domestic animals.

According to a preferred embodiment of the methods for controlling phytophagous organisms and pests of the present invention, one or more microorganisms containing dsRNA can be produced and used for protecting the plant against one or more species of insects or molluscs contemporaneously, or for protecting environments from infestations of harmful organisms. In this way, it is possible to obtain products with two or more types of dsRNA and thus act against various harmful species which co-exist in the same environment. A use of this type of product on plants, for example, would provide protection from the attack of both phytophagous organisms which prevalently feed on leaves, and also organisms which feed on bulbs or roots. Although preserving the specific resistance characteristics to single species of harmful organisms, plants can also be protected from various harmful species, even extremely different from each other, i.e. belonging to groups which are phylogenetically very distant from each other.

The present invention will now be described illustratively but non-limitedly, according to its preferred embodiments with particular reference to the figures of the enclosed drawings, in which:
figure 1 shows the sequences of the cDNAs encoding the GPCR receptors: AlstCR (SEQ. ID NO:1 and SEQ: ID NO.2) and AKHR (SEQ. ID NO:3 and SEQ. ID NO:4) cloned by *S. littoralis;*
figure 2 shows the RT-PCR analysis at different times of *E.coli* microorganisms expressing the dsRNA (corresponding to the DHR receptor) sprayed on tobacco leaves: the result provides some indications on the stability, and therefore effectiveness of the product with time;
figure 3, panels A-C, shows the graphs relating to the mortality percentage of *Spodoptera littoralis* larvae fed with the artificial diet containing 5,000 ppm of product which specifically inhibits the functionality of the receptors AlstCR, DHR and Oct/TyrR.

For illustrative and non-limiting purposes of the present invention, the production of bacteria of *E.coli* expressing the dsRNA corresponding to a part of or whole sequence of the GPCR receptors of *Spodoptera,* AlstCR, DHR and Oct/TyrR, is described hereunder. Once produced, the bacteria were administered to the larvae together with leaves starting from different stages in order to evaluate their toxic effect and measure the mortality rate.

### EXAMPLE 1: Transformation of E.coli bacteria with the plasmid containing the GPCR gene of Spodoptera littoralis.

### MATERIALS AND METHODS

### Selection of the GPCR target receptors

The receptors of *S. littoralis* selected as target of the present embodiment of the invention are:
1. AlstCR (putative receptor of allatostatin C): this GPCR is of fundamental importance as it binds allatostatins, neuropeptides whose main function is to inhibit the synthesis of the juvenile hormone on the part of the "corpus allatum". Allatostatins are not only involved in the metamorphosis process and ecdysis (Weaver et al., 1994), but are also important for regulating smooth muscle contractions of the intestines and in the development and functionality of the ovaries in adults (Aguilar et al., 2003; Meyering-Vos et al., 2006);
2. DHR (receptor of the diuretic hormone DH): important for regulating the diuresis (Johnson *et al*., 2004) and the water balance of the whole body of the insect, during both the larval and adult stage;
3. PBAN-R (receptor of the activation neuropeptide of the biosynthesis of pheromones, PBAN) : it binds a small peptide which promotes the synthesis and the release of sexual pheromones (Rafaeli *et al*., 2007):
4. LGR1 Leucine-Rich Repeat-Containing G Protein-Coupled Receptor): belongs to the group of G protein-coupled receptors containing Leucine-rich repeats (LGR) and exerts a fundamental role in reproduction (Nishi *et al*., 2000);
5. Oct/TyrR (putative receptor of octopamine and tyramine): it is capable of interacting with neuro-transmitters such as octopamine and tyramine and is therefore important in neuromodulation process in sensorial and secretory organs (Farooqui, 2007);
6. OR83b (receptor of the family of olfactory receptors): unlike other members of the family of olfactory receptors (OR), which are only expressed in small sub-populations of sensorial olfactory neurons, OR83b is expressed in almost all neurons of the feeler (Neuhaus *et al*., 2005). Rather than having a direct role in the olfactory function, it interacts with the conventional OR members and is essential for their diffusion from the cell bodies to the sensorial cilia where the interaction with the odorant molecules takes place. For the general role it exerts in the olfactory system, OR83b is certainly of fundamental importance for the perception of odours and therefore for the nutrition of the insect.

For effecting the RNAi experiments, all the sequences encoding the GPCR were amplified by the cDNA of *Spodoptera* with specific primers, with the exception of the genes encoding AlstCR (SEQ.ID NO:1) and AKHR (SEQ.ID NO:3) which were previously cloned by 5' and 3' RACE-PCR using degenerated primers.

### Isolation and cloning of the selected receptors.

The sequences encoding the receptors AlstCR and AKHR were isolated using the cDNA retro-transcribed from the total RNA extracted from *S*. *littoralis,* using degenerated primers deriving from comparison with regions preserved among these genes in the various insect species.

The following degenerated primers were used for the gene of AlstCR:
1. 1Fw: GA(AG)TG(TC)TT(TC)(TC)T(AGCT)AT(ATC)GG(ATGC) (SEQ ID NO:5);
2. 1Rv: (ATGC) GC (AG) CA (ATGC) GT (AG) CA (ATGC) GC (TC) TT (SEQ ID NO:6).
   The following degenerated primers were used for the gene of AKHR:
3. 1Fw: GACCTGATGTGC(AC)G(AC)(AG)TCATG (SEQ ID NO:7)
4. 1Rv: GTC(AG)ATCCA(AG)TACCACAG(AG)CA (SEQ ID NO:8).

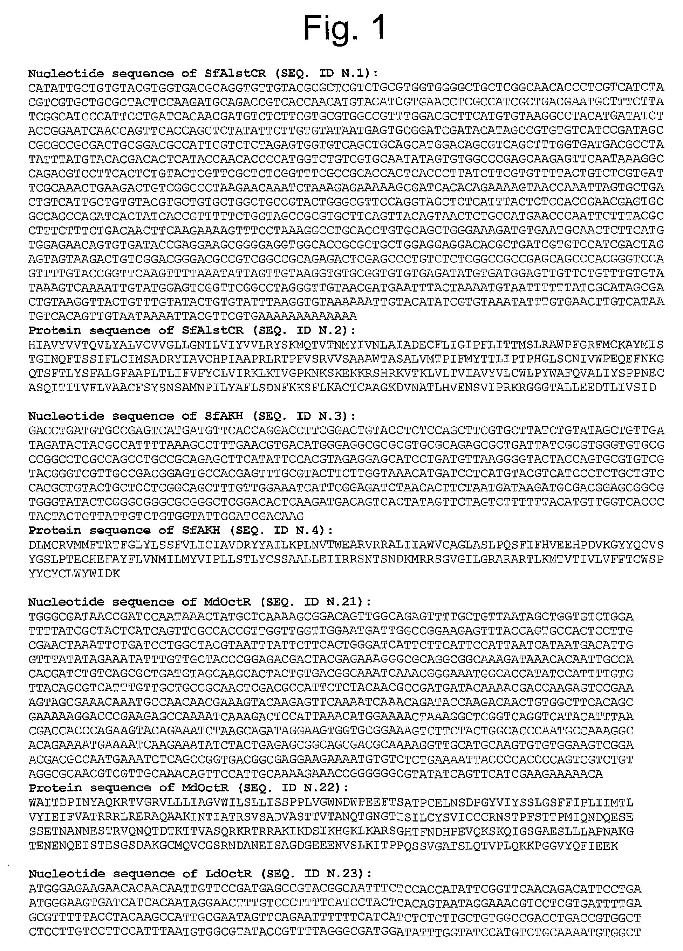

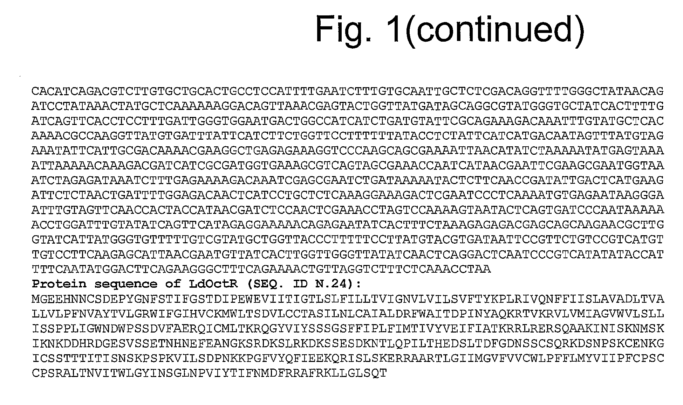

The PCR products obtained (corresponding to portions of sequence of these receptors) were sequenced and used for designing specific primers for the experiments of 5' and 3' RACE. The sequences isolated were cloned in the plasmid pCR^{®} 2.1-TOPO^{®} (*Invitrogen*) and sequenced (Figure 1).

For the other receptors (DHR, PBANR, LGR1, Oct/TyrR, OR83b) on the basis of the expression data available in literature, the sequences were isolated using different tissues and growth stages of the insect. In particular, the gene encoding the receptor LGR was amplified from eggs, whereas DHR from V-stage larvas; the genes encoding the receptors Oct/TyrR and OR83b were amplified by RNA extracted from the head of individual adults; V-stage larvas, on the other hand, were used for PBAN-R.

Once cloned in the vector pCR^{®} 2.1-TOPO^{®}, all the genes encoding the receptors were transferred by digestion into the vector L4440. For the genes of AlstCR and DHR, the restriction enzymes KpnI and XhoI were used; for that of PBANR, EcoRI was used, whereas for those of LGR, Oct/TyrR and OR83b, SacI and XhoI were used. The ligation reactions between the genes of the receptors (200 ng) and the vector (300 ng) were effected in a volume of 20 ul at 16°C using 20 units of T4 DNA ligase (BioLabs). The vector L4440 is a modified version of the plasmid pBluescript containing at both ends of the polilinker the promoter for T7 RNA polymerase, capable of transcribing the gene in both directions. In this way, two transcripts are produced, one "sense" the other "antisense", which once coupled, form a single dsRNA.

### Transformation and growth of the bacterial cultures:

50 ng of recombinant plasmid, each containing the sequence of the receptor selected or part of it, were transformed into cells of *E*. *coli* belonging to the strain HT115 (DE3). The cells were kept in ice for 30 minutes. The thermal shock was effected by keeping the cells for 1 minute at 37°C and then 2 minutes in ice. 1 ml of YEP was added to the cells. After 1 hour of growth at 37°C, the cells were plated on YEP-agar containing tetracycline 12.5 ug/ul and ampicillin 100 ug/ul. The plate was left overnight at 37°C. A transformed colony was inoculated in YEP containing tetracycline 12.5 ug/ul and ampicillin 100 ug/ul and was grown for 18-20 hours. The following day the cells were diluted in 500 ml of YEP containing antibiotics and grown until reaching an OD600 (optical density measured at 600 nm) equal to 0.4. The specific gene expression was induced with IPTG 0.4 mM. After induction, the cells were left to grow all night at 37°C. The cells were centrifuged at 6,000 rpm for 15 minutes. The pellet obtained was frozen and then lyophilized. After the lyophilization, the pellet obtained was boiled for about 15 minutes to deactivate the bacteria. For the tests on insects, the pellet containing the dsRNA was resuspended in water and used at the selected concentration.

### Tests with larvae of S. littoralis:

Larvae of *S. littoralis* of different stages were used for the biological tests. A number varying from 70 to 200 larvae were used for each experiment. After the eggs had hatched, the larvae were first kept together in a single container in the presence of food, then isolated starting from stage I, II or III. Each larva was placed in a container having a diameter of 6 cm into which about 3 g of food containing from 5,000 to 15,000 ppm of bacterial lyophlized product, was introduced daily. Every 2 days, the food was substituted with fresh food, whereas every day the number of dead and alive larvae were counted to calculate the mortality percentage of the populations with time. The food used for the larvae, "pabulum", had the following composition: for each 1.14 l of water, 33.75 g of agar, 47.27 g of wheat germs, 67.5 g of beer yeast, 189 g corn flour, 6.75 g of ascorbic acid, 1.7 g benzoic acid and 1.35 g of nipagin, are added.

### RESULTS

Once the *E. coli* bacteria had been transformed with the plasmid L4440 for the expression of the dsRNA, they were lyophilized and used for verifying the effect of the dsRNA contained therein on the vitality of the larvae of *S. littoralis.*

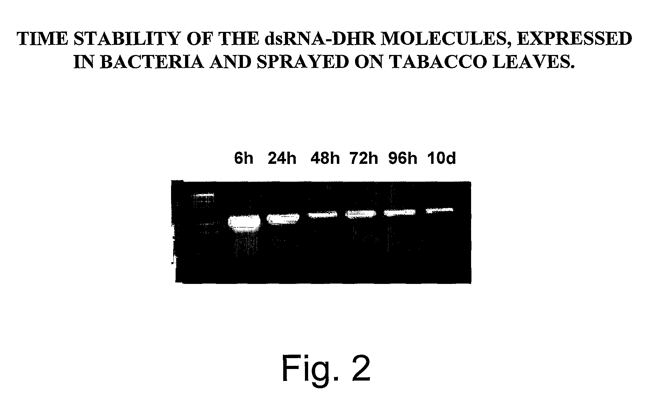

Before administering the dsRNA contained in the bacteria to the larvas, its stability with time was verified to ensure that the larvas are fed each day with a product containing dsRNA molecules which are intact and consequently functioning for the gene silencing. Bacteria containing dsRNA molecules corresponding to the DHR receptor, suitably deactivated, were therefore sprayed onto tobacco leaves removed from the plant at a concentration of 5,000 ppm. The dsRNA was re-isolated at intervals of 24 hours by the extraction of RNA and amplified with specific primers which allow the whole of its gene sequence to be visualized. As shown in Figure 2, even after 10 days, the presence of dsRNA molecules can be detected on the leaf, even if the quantity is much lower than that observed only a few hours after the treatment. This result confirms that the product, object of this invention can be used, as it is stable with time and moreover does not persist for excessively long times.

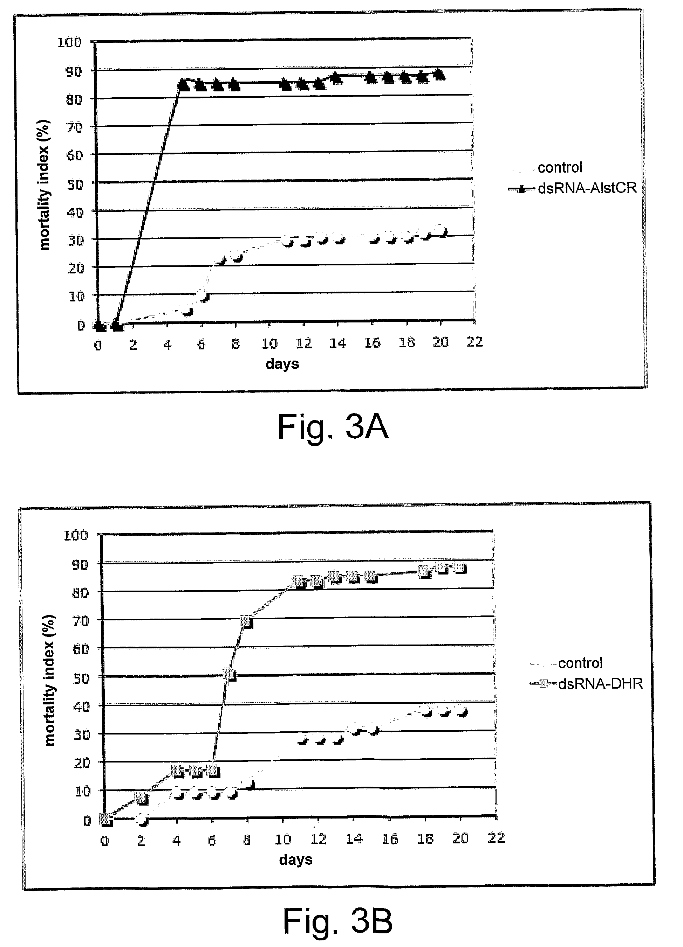

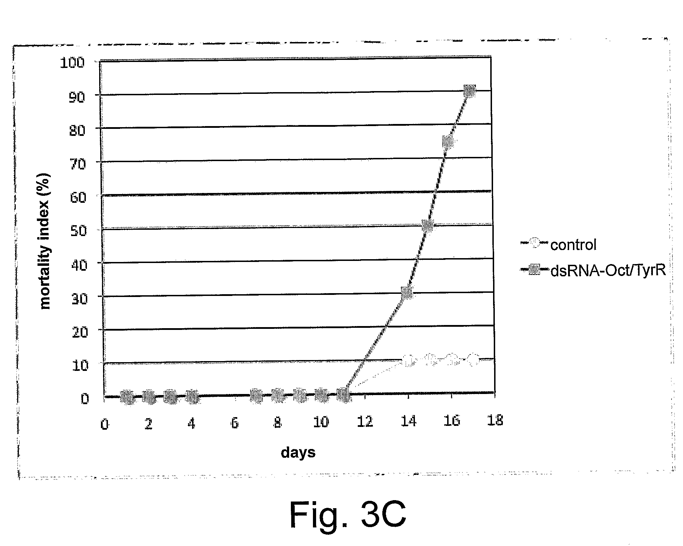

Once the stability of the dsRNA had been verified, a series of experiments were prepared, in which larvae starting from stage I were fed with "pabulum" mixed with 5,000 ppm of bacteria containing dsRNA corresponding to the gene of AlstCR. In these experiments, about 200 larvae were used, fed with bacteria expressing dsRNA right from the first stage after the hatching of the eggs, and compared with the same number of control larvae, fed however with a diet containing bacteria not expressing dsRNA. Figure 3 (graph A) shows the graph of the trend of the mortality rate of a population of control larvae with respect to a population fed with bacteria expressing dsRNA. Even after a few days, the effect of the dsRNA on the vitality of the larvae is evident: after 20 days (shortly before the metamorphosis), the mortality rate of the population of larvae fed with dsRNA reached 90%, whereas that of the control larvae was about 30%. It is consequently evident that the population of larvae fed with the diet containing the product of the invention shows a much higher mortality percentage with respect to the larvae fed with the control diet.

In a second series of experiments, mortality rates were measured in populations of larvae (each of 70 individuals) fed starting from stage III with 10,000 ppm of bacteria containing dsRNA corresponding to the gene of the DHR receptor, and again compared with control larvae fed with a diet containing bacteria without dsRNA. Figure 3 (graph B) shows the results of this experiment, which, also in this case, clearly indicate the significant effect of dsRNA on the vitality of the larvae: after about 20 days, the mortality rate is about 90% in the population treated, against 40% of the control population.

Finally, a third experiment was performed, also testing the effect of dsRNA corresponding to the gene of the receptor of octopamine/tyramine (Oct/TyrR) on larvae starting from stage III, analogously to what was done for the DHR receptor. The results, shown in Figure 3 (graph C) indicate that also for the Oct/TyrR receptor, the mortality rate of the larvae fed with dsRNA reaches 90% after 17 days, against only 10% of the control population fed with bacteria not expressing dsRNA.

All three experiments described, effected on three different types of dsRNA (i.e. corresponding to three different GPCR targets), clearly suggest that the technology in question, which the applicant intends to protect, is valid for the control of phytophagous organisms and can also be extended to all pests harmful both for agriculture and for housing, for human or animal use.

### EXAMPLE 2: Transformation of E.coli bacteria with the plasmid containing the gene of the octopamine receptor of Musca domestica.

### MATERIALS AND METHODS

### Selection of the GPCR target receptor

The receptor of *M*. *domestica* selected as target of the present embodiment of the invention is that of octopamine (MdOctR). It is in fact of fundamental importance in insects, as it is capable of interacting with neuro-transmitters such as octopamine and tyramine during all neuromodulation processes in sensorial and secretory organs (Farooqui, 2007).

### Isolation and cloning of the receptor selected

Part of the sequence encoding the receptor MdOctR was isolated using the cDNA retro-transcribed from the total RNA extracted from heads of adult individuals of *M.domestica,* using degenerated primers deriving from a comparison with regions preserved among these genes in the various insect species, as also in the case of the isolation of the sequences of *S.littoralis.* The degenerated primers used are the following:
1. 1Fw:
   TGGGC (ATCG) AT (ATC) AC (ATCG) GA (TC) CC (ATCG) AT (ATC) AA (TC) (SEQ ID NO: 25);
2. IRv:
   CA (ATCG) AC (ATCG) AC (AG) AA (ATCG) AC (ATCG) CCCAT (ATG) AT (SEQ ID NO: 26).

The PCR product obtained (corresponding to a portion of sequence of the receptor) was cloned in the plasmid pCR^{®} 2.1-TOPO^{®} (*Invitrogen*) and sequenced (Figure 1).

Once it had been cloned in the vector pCR^{®} 2.1-TOPO^{®} , the gene was transferred by digestion into the vector L4440, as done for the genes of *S. littoralis.*

### Transformation and growth of the bacterial culture

50 ng of recombinant plasmid, containing part of the sequence of the receptor MdOctR, was transformed into cells of *E. coli* belonging to the strain HT115 (DE3). The whole procedure used, from the transformation of the bacterial cells to the production of dsRNA, is identical to that adopted for the genes of *S. littoralis,* already amply described above.

### Tests with larvae of M. domestica:

Three day old larvae of *M*. *domestica* were used for the biological tests. 15 larvae were used for each experiment, which were placed in 50 ml containers containing about 10 g of artificial diet mixed with 2 ml of product dissolved in water, so as to have a final concentration of the product of about 3,000-5,000 ppm. At the end of the larval cycle, which lasts 6 days, the larvae were analyzed to calculate the mortality rate. The food used for the larvae has the following composition: 100 g of wheat bran and 20 g of powder milk are added to each 100 ml of demineralized water.

### RESULTS

Once the stability of the dsRNA had been verified, a series of experiments were prepared, in which the larvae, starting from the third day after the hatching of the eggs, were fed with an artificial diet mixed with 3,000-5,000 ppm of bacteria containing dsRNA corresponding to the gene of MdOctR. In these experiments, 15 larvae were used, fed with bacteria expressing dsRNA, and compared with the same number of control larvae, fed however with a diet containing bacteria not expressing dsRNA. The average mortality rate was calculated in the populations of larvae fed with bacteria expressing dsRNA and in those fed with bacteria not expressing dsRNA, 6 days after the hatching of the eggs, i.e. at the end of the larval cycle and prior to the pupal stage. The average mortality rate of the populations of larvae fed with dsRNA reached 34%, whereas that of the control larvae was only 13%. It is therefore evident that the larvae fed with the diet containing the product of the invention show a much high mortality percentage with respect to larvae fed with the control diet.

### EXAMPLE 3: Transformation of E.coli bacteria with the plasmid containing the gene of the octopamine receptor of Leptinotarsa decemlineata.

### MATERIALS AND METHODS

### Isolation and cloning of the receptor selected

Also for *Leptinotarsa decemlineata* the receptor selected as target of the present embodiment of the invention is that of octopamine (LdOctR).

Part of the sequence encoding the receptor LdOctR was isolated using the cDNA retro-transcribed from the total RNA extracted from heads of *L*. *decemlineata,* in the adult stage, using degenerated primers deriving from a comparison with regions preserved among these genes in the various insect species, as also in the case of the isolation of the sequences of *S.littoralis.* The degenerated primers used for this cloning are the same as those used for the cloning of the MdOctR receptor of M. domestica (SEQ. ID N. 25 and 26).

The PCR product obtained (corresponding to a portion of sequence of this receptor) was sequenced and used for designing specific primers for the experiments of 5' and 3' RACE. The sequence isolated, corresponding to the whole gene of the receptor, was cloned in the plasmid pCR^{®} 2.1-TOPO^{®} (*Invitrogen*) and sequenced (Figure 1).

Once it had been cloned in the vector pCR^{®} 2.1-TOPO^{®}, the gene was transferred by digestion into the vector L4440, as effected for the genes of *S*.

### littoralis.

### Transformation and growth of the bacterial culture:

50 ng of recombinant plasmid, containing the whole sequence of the receptor LdOctR, were transformed into cells of *E. coli* belonging to the strain HT115 (DE3). The whole procedure used, from the transformation of the bacterial cells to the production of dsRNA, is identical to that adopted for the genes of *S*. *littoralis* and *M*. *domestica,* already amply described above.

### Tests with larvae of L. decemlineata:

Larvae of *L*. *decemlineata* were used for the biological tests, starting from the first stage. About 20 larvae deriving from the hatching of eggs situated on leaves of potato plants were used for each experiment. After the hatching of the eggs, the larvae were fed with leaves of the control plants (treated with bacteria not containing dsRNA) and plants sprayed with an aqueous solution of the product (bacteria containing dsRNA) at a concentration of 15,000 ppm. The larvae were analyzed during the first and second stage, as far as the pupal stage, to calculate the mortality rate.

### RESULTS

Once the stability of the dsRNA had been verified, a series of experiments were prepared to calculate the effect of the product on the larvae of *Leptinotarsa,* during the various growth stages. Populations of 20 larvae were used in these experiments, fed with bacteria expressing dsRNA right from the first stage after the hatching of the eggs, and compared with the same number of control larvae, fed instead with a diet containing bacteria not expressing dsRNA. Although the experiments are still underway, some preliminary data indicate that also for *Leptinotarsa* the mortality rate of the larvae fed with the product of the invention is higher with respect to that of the larvae fed with the control diet.

### BIBLIOGRAPHY

- Aguilar R, Maestro JL, Vilaplana L, Pascual N, Piulachs MD, Bellés X (2003). Regul Pept. Oct 15; 115(3):171-7.
- Baum JA, Bogaert T, Clinton W, Heck GH, Feldman P, Ilagan O, Johnson S, Plaetinck G, Munyikwa T, Pleau M, vaughn TY and Roberts J (2007). Nature Biotech, 25 (11): 1322-1326.
- Brantl, S. (2002) Biochimica et Biophysica Acta. 1575:15-25.
- Farooqui T (2007). Neurochem Res. 32: 1511-1529.
- Hamilton, A.J. and Baulcombe, D.C. (1999). Science 286: 950-952.
- Hammond, S.M. Boettcher, S., Caudy, A., Kobayashi, R. and Hannon, G. (2001). Science 293: 1146-1150.
- Hill S.J. (2006). Br J Pharmacol. 147 (S1): S27-S37.
- Johnson EC, Bohn LM and Taghert PH (2004). Journal of Exper Biol, 207 : 743-748.
- Meyering-Vos M, Merz S, Sertkol M, Hoffmann KH (2006). Insect Biochem Mol Biol. Jun;36(6):492-504.
- Neuhaus EM, Gisselmann G, Zhang W, Dooley R, Stortkuhl K Hatt H (2005). Nature Neurosc, 8 (1) : 15-17.
- Nishi S, Hsu SY, Zell K and Hsueh JW (2000). Endocrinology, 141 (11): 4081-4090.
- Rafaeli A, Bober R, Becker L, Choi MY, Fuerst EJ and Jurenka R (2007). Insect Molec Biol, 16 (3): 287-293.
- Tomoyasu, Y. and Denell, R. E. (2004). Dev. Genes Evol. 214:575-578.
- Weaver RJ, Freeman ZA, Pickering MG, Edwards JP. (1994). Comp. Biochem. Physiol. C 107:119-27.

## Claims

1. Use of inactivated transgenic microorganisms transformed with at least one expression vector comprising a cDNA sequence encoding for a GPCR receptor which is vital for a phytophagous animal or pest, said expression vector being capable of expressing dsRNA corresponding to said cDNA sequence, said receptor being selected from the group consisting of allatostatin receptor, diuretic hormone receptor, octopamine/tyramine receptor, alone or in combination with other strains of microorganisms previously transformed with a cDNA sequence encoding for another GPCR receptor which is vital for a different phytophagous animal or pest, as mixture of pesticides against larvae and/or adult insects;
wherein said microorganisms are transformed with a cDNA sequence encoding for a GPCR receptor which is vital for *Spodoptera littoralis* and having a nucleotide sequence selected from the group consisting of allatostatin C receptor AlstCR (SEQ ID NO:1), diuretic hormone receptor, DHR (SEQ ID NO:11), receptor of octopamine/tyramine, Oct/TyrR (SEQ ID NO:15), as pesticide for the eradication of the phytophagous *Spodoptera littoralis,* or
wherein said microorganisms are transformed with a cDNA sequence encoding a GPCR receptor which is vital for *Musca domestica* having the nucleotide sequence (SEQ ID NO:21) encoding the receptor of octopamine having SEQ ID NO:22, as pesticide for the eradication of the dipteran *Musca domestica;* or
wherein said microorganisms are transformed with a cDNA sequence encoding a GPCR receptor which is vital for *Leptinotarsa decemlineata* having the nucleotide sequence (SEQ ID NO:23) encoding the receptor of octopamine having SEQ ID NO:24, as pesticide for the eradication of the coleopteran *Leptinotarsa decemlineata*.

2. Use according to claim 1, wherein said microorganisms are selected from the group consisting of bacteria, yeasts, fungi, protozoa, insect cells, mammalian cells or plant cells.

3. Use according to anyone of the claims 1-2, wherein said microorganisms are selected from *E.coli, Saccharomyces,* or *Pichia*.

4. Method for obtaining transgenic microorganisms suitable for eliminating the attack of one or more phytophagous animals or pests, containing at least one dsRNA able to inhibit the functionality of a GPCR receptor, comprising the following steps:
a) isolation of the cDNA nucleotide sequence encoding for GPCR receptor , carrying out important functions in the phytophagous animal or pest of interest, said receptor being selected from the group consisting of allatostatin, diuretic hormone, octopamine/tyramine;
b) construction of an expression vector or recombinant plasmid comprising the cDNA nucleotide sequence encoding for a GPCR receptor, as determined in step a);
c) transfer of said vector or recombinant plasmid into the cells of a microorganism selected from bacteria, yeasts, fungi, protozoa, or in eukaryotic cell selected from insect cells, mammalian cells or plant cells for dsRNA expression;
d) growth of the microorganisms or eukariotic cells culture expressing dsRNA, until reaching dense cultures;
e) spinning of the cell culture to eliminate culture medium, washing of the obtained cell pellet and lyophilization to eliminate the liquid component;
f) heat inactivation of the microorganisms by heat or by other physical procedures;wherein said microorganisms are transformed with a cDNA sequence encoding for a GPCR receptor which is vital for *Spodoptera littoralis* and having a nucleotide sequence selected from the group consisting of allatostatin C receptor AlstCR (SEQ ID NO:1), diuretic hormone receptor, DHR (SEQ ID NO:11), receptor of octopamine/tyramine, Oct/TyrR (SEQ ID NO:15), as pesticide for the eradication of the phytophagous *Spodoptera littoralis,* or
wherein said microorganisms are transformed with a cDNA sequence encoding a GPCR receptor which is vital for *Musca domestica* having the nucleotide sequence (SEQ ID NO:21) encoding the receptor of octopamine having SEQ ID NO:22, as pesticide for the eradication of the dipteran *Musca domestica;* or
wherein said microorganisms are transformed with a cDNA sequence encoding a GPCR receptor which is vital for *Leptinotarsa decemlineata* having the nucleotide sequence (SEQ ID NO:23) encoding the receptor of octopamine having SEQ ID NO:24, as pesticide for the eradication of the coleopteran *Leptinotarsa decemlineata*.

5. A pesticide composition comprising inactivated transgenic microorganisms, as defined according to claims 1-3, as active ingredient to be used at a concentration ranging from 1000 to 30000 ppm in aqueous solution, optionally in presence of excipients and/or enhancers used in the agriculture field or in pesticide formulations.

6. A pesticide composition according to claim 5, wherein said microorganisms can be represented by different strains belonging to the same species, each strain transformed with a cDNA encoding for a GPCR which is vital for different phytophagous animal and/or pest.

7. A pesticide composition, according to anyone of the claims 5-6, formulated as spray, powder, aqueous suspension, lyophilized preparation, paste.

8. Method to control and/or to eliminate one or more phytophagous animal or pest in the adult or larval stage for the treatment of agronomical, ornamental and environmentally interesting plants, comprising spraying the pesticide composition, as defined according to any of the claims 5-7, on the leaves or on the stem of said plants or on the ground.

9. Method for the control and/or elimination of pests in the adult or larval stage from an environment, comprising the application of a pesticide composition, as defined according to any of the claims 5-7, as powder, solution or by spraying on a surface selected from wood, cloth, soil, water ponds, animal excrements.

10. Method according to anyone of the claims 8 or 9, wherein said microorganisms can be used alone or in combination with other strains of microorganisms transformed with cDNA encoding for a GPCR receptor which is vital for a different phytophagous animal or pest agent in the adult and/or larval stage.

## Patentansprüche

1. Verwendung von inaktivierten transgenen Mikroorganismen, transformiert mit mindestens einem Expressionsvektor, umfassend eine cDNA-Sequenz, die für einen GPCR-Rezeptor kodiert, der für ein pflanzenfressendes Tier oder einen Schädling lebensnotwendig ist, wobei der Expressionsvektor in der Lage ist, dsRNA entsprechend der cDNA-Sequenz zu exprimieren, wobei der Rezeptor ausgewählt ist aus der Gruppe bestehend aus Allatostatin-Rezeptor, diuretischer Hormon-Rezeptor, Optopamin-/Tyramin-Rezeptor, allein oder in Verbindung mit anderen Stämmen von Mikroorganismen, die zuvor mit einer cDNA-Sequenz transformiert wurden, die für einen anderen GPCR-Rezeptor kodiert, der für ein anderes pflanzenfressendes Tier oder einen Schädling lebensnotwendig ist, als Gemisch von Pestiziden gegen Larven und/oder erwachsene Insekten;
wobei die Mikroorganismen mit einer cDNA-Sequenz transformiert sind, die für einen GPCR-Rezeptor kodiert, der für *Spodoptera littoralis* lebensnotwendig ist und eine Nukleotidsequenz aufweist, ausgewählt aus der Gruppe bestehend aus Allatostatin-C-Rezeptor AlstCR (SEQ ID NO:1), diuretischer Hormon-Rezeptor, DHR (SEQ ID NO:11), Octopamin-/Tyramin-Rezeptor, Oct/TyrR (SEQ ID NO:15), als Pestizid zur Ausrottung der pflanzenfressenden *Spodoptera littoralis;* oder
wobei die Mikroorganismen transformiert sind mit einer cDNA-Sequenz, die für einen GPCR-Rezeptor kodiert, der für *Musca domestica* lebensnotwendig ist und die Nukleotidsequenz (SEQ ID NO:21) aufweist, die für den Octopamin-Rezeptor mit SEQ ID NO:22 kodiert, als Pestizid zur Ausrottung der dipteranen *Musca domestica;* oder
wobei die Mikroorganismen transformiert sind mit einer cDNA-Sequenz, die für einen GPCR-Rezeptor kodiert, der für *Leptinotarsa decemlineata* lebensnotwendig ist und die Nukleotidsequenz (SEQ ID NO:23) aufweist, die für den Octopamin-Rezeptor mit SEQ ID NO:24 kodiert, als Pestizid zur Ausrottung der coleopteranen *Leptinotarsa decemlineata*.

2. Verwendung nach Anspruch 1, wobei die Mikroorganismen ausgewählt sind aus der Gruppe bestehend aus von Bakterien, Hefen, Pilzen, Protozoen, Insektenzellen, Säugetierzellen oder Pflanzenzellen.

3. Verwendung nach einem der Ansprüche 1 bis 2, wobei die Mikroorganismen ausgewählt sind aus *E.coli, Saccharomyces* oder *Pichia*.

4. Verfahren zur Gewinnung von transgenen Mikroorganismen, die geeignet sind, die Angriffe von einem oder mehreren pflanzenfressenden Tieren oder Schädlingen abzuwehren, enthaltend mindestens eine dsRNA, die in der Lage ist, die Funktionsfähigkeit eines GPCR-Rezeptors zu hemmen, umfassend folgende Schritte:
a) Isolieren der cDNA-Nukleotidsequenz, die für GPCR-Rezeptor kodiert, der wichtige Funktionen im betreffenden pflanzenfressenden Tier oder Schädling ausführt, wobei der Rezeptor ausgewählt ist aus der Gruppe bestehend aus Allatostatin, diuretisches Hormon, Octopamin/Tyramin;
b) Aufbauen eines Expressionsvektors oder rekombinanten Plasmids umfassend die cDNA-Nukleotidsequenz, die für einen GPCR-Rezeptor kodiert, wie in Schritt a) dargelegt ist;
c) Übertragen des Vektors oder rekombinanten Plasmids in die Zellen eines Mikroorganismus, ausgewählt aus Bakterien, Hefen, Pilzen, Protozoen, oder in eukaryotische Zellen, ausgewählt aus Insektenzellen, Säugetierzellen oder Pflanzenzellen zur dsRNA-Expression;
d) Züchten der Mikroorganismen oder eukaryotischen Zellkulturen, die die dsRNA exprimieren, bis zum Erreichen von dichten Kulturen;
e) Zentrifugieren der Zellkulturen zur Beseitigung des Kulturmediums, Waschen des erhaltenen Zellpellets und Lyophilisieren zur Beseitigung des flüssigen Anteils;
f) Hitzeinaktivieren der Mikroorganismen durch Hitze oder sonstige physikalische Verfahren;
wobei die Mikroorganismen mit einer cDNA-Sequenz transformiert sind, die für einen GPCR-Rezeptor kodiert, der für *Spodoptera littoralis* lebensnotwendig ist und eine Nukleotidsequenz aufweist, ausgewählt aus der Gruppe bestehend aus Allatostatin-C-Rezeptor AlstCR (SEQ ID NO:1), diuretischer Hormon-Rezeptor, DHR (SEQ ID NO:11), Octopamin-/Tyramin-Rezeptor, Oct/TyrR (SEQ ID NO:15), als Pestizid zur Ausrottung der pflanzenfressenden *Spodoptera littoralis;* oder
wobei die Mikroorganismen transformiert sind mit einer cDNA-Sequenz, die für einen GPCR-Rezeptor kodiert, der für *Musca domestica* lebensnotwendig ist und die Nukleotidsequenz (SEQ ID NO:21) aufweist, die für den Octopamin-Rezeptor mit SEQ ID NO:22 kodiert, als Pestizid zur Ausrottung der dipteranen *Musca domestica;* oder
wobei die Mikroorganismen transformiert sind mit einer cDNA-Sequenz, die für einen GPCR-Rezeptor kodiert, der für *Leptinotarsa decemlineata* lebensnotwendig ist und die Nukleotidsequenz (SEQ ID NO:23) aufweist, die für den Octopamin-Rezeptor mit SEQ ID NO:24 kodiert, als Pestizid zur Ausrottung der coleopteranen *Leptinotarsa decemlineata*.

5. Pestizide Zusammensetzung, umfassend inaktivierte transgene Mikroorganismen wie in den Ansprüchen 1 bis 3 definiert, zu verwenden als Wirkstoff in einer Konzentration zwischen 1.000 und 30.000 ppm in wässriger Lösung, wahlweise in Gegenwart von Trägerstoffen und/oder Verstärkern, die im Bereich der Landwirtschaft oder in Pestizidformulierungen verwendet werden.

6. Pestizide Zusammensetzung nach Anspruch 5, wobei die Mikroorganismen durch verschiedene, zur selben Spezies gehörende Stämme vertreten sein können, wobei jeder Stamm mit einer cDNA transformiert ist, die für einen GPCR kodiert, der für ein anderes pflanzenfressendes Tier und/oder Schädling lebensnotwendig ist.

7. Pestizide Zusammensetzung nach einem der Ansprüche 5 bis 6, formuliert als Spray, Pulver, wässrige Lösung, lyophilisierte Präparation oder Paste.

8. Verfahren zur Kontrolle und/oder Beseitigung eines oder mehrerer pflanzenfressender Tiere oder Schädlinge im Erwachsenen- oder Larvenstadium zur Behandlung von Nutz-, Zier- oder umweltbezogen wichtigen Pflanzen, umfassend das Versprühen der pestiziden Zusammensetzung nach einem der Ansprüche 5 bis 7 auf den Blättern oder Stängeln der Pflanzen oder auf dem Boden.

9. Verfahren zur Kontrolle und/oder Beseitigung von Schädlingen im erwachsenen Stadium oder Larvenstadium in einer Umgebung, umfassend die Anwendung einer pestiziden Zusammensetzung nach einem der Ansprüche 5 bis 7 als Pulver, Lösung oder Versprühen auf einer Fläche, ausgewählt aus Holz, Stoff, Erdreich, Wasserflächen oder tierischen Exkrementen.

10. Verfahren nach einem der Ansprüche 8 oder 9, wobei die Mikroorganismen allein oder in Kombination mit anderen Stämmen von Mikroorganismen verwendet werden können, die mit einer cDNA transformiert sind, die für einen GPCR-Rezeptor kodiert, der für ein anderes pflanzenfressendes Tier oder einen Schädling im Erwachsenen- und/oder Larvenstadium lebensnotwendig ist.

## Revendications

1. Utilisation de micro-organismes transgéniques inactivés transformés avec au moins un vecteur d'expression comprenant une séquence d'ADNc codant pour un récepteur GPCR qui est vital pour un parasite ou animal phytophage, ledit vecteur d'expression étant capable d'exprimer des ARNds correspondant à ladite séquence d'ADNc, ledit récepteur étant sélectionné à partir du groupe comprenant un récepteur allatostatine, un récepteur hormonal diurétique, un récepteur octopamine/tyramine, seul ou en combinaison avec d'autres souches de micro-organismes préalablement transformés avec une séquence d'ADNc codant pour un autre récepteur GPCR qui est vital pour un parasite ou animal phytophage différent, comme un mélange de pesticides contre des larves et/ou des insectes adultes ; dans laquelle lesdits micro-organismes sont transformés avec une séquence d'ADNc codant pour un récepteur GPCR qui est vital pour *Spodoptera littoralis* et ayant une séquence nucléotide sélectionnée à partir du groupe comprenant le récepteur C allatostatine AlstCR (SEQ ID N° 1), le récepteur hormonal diurétique DHR (SEQ ID N° 11), un récepteur octopamine/tyramine Oct/TyrR (SEQ ID N° 15), comme pesticide pour l'éradication du phytophage *Spodoptera littoralis,* ou
dans laquelle lesdits micro-organismes sont transformés avec une séquence d'ADNc codant pour un récepteur GPCR qui est vital pour *Musca Domestica* ayant la séquence nucléotide (SEQ ID N° 21) codant le récepteur d'octopamine ayant la SEQ ID N° 22, comme pesticide pour l'éradication de la *Musca Domestica* de l'ordre des diptères ; ou
dans laquelle lesdits micro-organismes sont transformés avec une séquence d'ADNc codant pour un récepteur GPCR qui est vital pour *Leptinotarsa decemlineata* ayant la séquence nucléotide (SEQ ID N° 23) codant le récepteur d'octopamine ayant la SEQ ID N° 24, comme pesticide pour l'éradication de la *Leptinotarsa decemlineata* de l'ordre des coléoptères.

2. Utilisation selon la revendication 1, dans laquelle lesdits micro-organismes sont sélectionnés à partir du groupe comprenant des bactéries, levures, champignons, protozoaires, cellules d'insectes, cellules de mammifères ou cellules végétales.

3. Utilisation selon l'une quelconque des revendications 1 à 2, dans laquelle lesdits micro-organismes sont sélectionnés à partir des *E.coli, Saccharomyces* ou *Pichia*.

4. Procédé pour obtenir des micro-organismes transgéniques adaptés pour éliminer l'attaque d'un ou plusieurs parasites ou animaux phytophages, contenant au moins un ARNds capable d'inhiber la fonctionnalité d'un récepteur GPCR, comprenant les étapes suivantes :
a) l'isolation de la séquence nucléotide d'ADNc codant pour un récepteur GPCR, exécutant des fonctions importantes dans l'animal ou le parasite phytophage d'intérêt, ledit récepteur étant sélectionné à partir du groupe comprenant allatostatine, hormone diurétique, octopamine/tyramine ;
b) la construction d'un vecteur d'expression ou plasmide recombinant comprenant la séquence nucléotide d'ADNc codant pour un récepteur GPCR, telle que déterminée dans l'étape a) ;
c) le transfert dudit vecteur ou plasmide recombinant dans les cellules d'un micro-organisme sélectionné à partir de bactéries, levures, champignons, protozoaires, ou dans une cellule eucaryote sélectionnée parmi des cellules d'insectes, cellules de mammifères ou cellules végétales pour l'expression d'ARNds ;
d) la croissance de la culture de micro-organismes ou cellules eucaryotes exprimant l'ARNds, jusqu'à l'obtention de cultures denses ;
e) la centrifugation de la culture cellulaire pour éliminer le milieu de culture, le lavage du culot de cellules obtenu et la lyophilisation pour éliminer le composant liquide ;
f) l'inactivation thermique des micro-organismes par la chaleur ou par d'autres procédures physiques ; dans lequel lesdits micro-organismes sont transformés avec une séquence d'ADNc codant pour un récepteur GPCR qui est vital pour *Spodoptera littoralis* et ayant une séquence nucléotide sélectionnée à partir du groupe comprenant le récepteur C allatostatine AlstCR (SEQ ID N° 1), récepteur hormonal diurétique DHR (SEQ ID N° 11), un récepteur octopamine/tyramine Oct/TyrR (SEQ ID N° 15), comme pesticide pour l'éradication du phytophage *Spodoptera littoralis,* ou
dans lequel lesdits micro-organismes sont transformés avec une séquence d'ADNc codant pour un récepteur GPCR qui est vital pour *Musca Domestica* ayant la séquence nucléotide (SEQ ID N° 21) codant le récepteur d'octopamine ayant la SEQ ID N° 22, comme pesticide pour l'éradication de la *Musca Domestica* de l'ordre des diptères ; ou
dans lequel lesdits micro-organismes sont transformés avec une séquence d'ADNc codant pour un récepteur GPCR qui est vital pour *Leptinotarsa decemlineata* ayant la séquence nucléotide (SEQ ID N° 23) codant le récepteur d'octopamine ayant la SEQ ID N° 24, comme pesticide pour l'éradication de la *Leptinotarsa decemlineata* de l'ordre des coléoptères.

5. Composition pesticide comprenant des micro-organismes transgéniques inactivés, tels que définis selon les revendications 1 à 3, comme ingrédient actif à utiliser à une concentration dans la plage de 1000 à 30000 ppm en solution aqueuse, facultativement en présence d'excipients et/ou d'amplificateurs utilisés dans le domaine de l'agriculture ou dans des préparations pesticides.

6. Composition pesticide selon la revendication 5, dans laquelle lesdits micro-organismes peuvent être représentés par différentes souches appartenant aux mêmes espèces, chaque souche étant transformée avec un ADNc codant pour un récepteur GPCR qui est vital pour un parasite et/ou animal phytophage différent.

7. Composition pesticide selon l'une quelconque des revendications 5 à 6, se présentant sous forme de pulvérisation, poudre, suspension aqueuse, préparation lyophilisée, pâte.

8. Procédé pour contrôler et/ou éliminer un ou plusieurs parasites ou animaux phytophages au stade adulte ou larvaire pour le traitement de plantes agronomiques, ornementales et intéressantes du point de vue environnemental, comprenant la pulvérisation de la composition pesticide, telle que définie selon l'une quelconque des revendications 5 à 7, sur les feuilles ou sur la tige desdites plantes ou sur le sol.

9. Procédé pour le contrôle et/ou l'élimination de parasites au stade adulte ou larvaire d'un environnement, comprenant l'application d'une composition pesticide, telle que définie selon l'une quelconque des revendications 5 à 7, en tant que poudre, solution ou par pulvérisation sur une surface sélectionnée parmi du bois, du tissu, le sol, des étangs, des excréments d'animaux.

10. Procédé selon l'une quelconque des revendications 8 ou 9, dans lequel lesdits micro-organismes peuvent être utilisés seuls ou en combinaison avec d'autres souches de micro-organismes transformés avec une séquence d'ADNc codant pour un récepteur GPCR qui est vital pour un agent parasite ou animal phytophage différent au stade adulte et/ou larvaire.
